(19) **Europäisches Patentamt**
**European Patent Office**
**Office européen des brevets**

(11) **EP 4 397 743 A1**

(12) **EUROPEAN PATENT APPLICATION**

(43) Date of publication:
**10.07.2024 Bulletin 2024/28**

(21) Application number: **23382002.6**

(22) Date of filing: **03.01.2023**

(51) International Patent Classification (IPC):
**C12M 1/00** *(2006.01)*    **C12M 1/42** *(2006.01)*

(52) Cooperative Patent Classification (CPC):
**C12M 35/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
  • **Consejo Superior de Investigaciones Científicas (CSIC)**
    **28006 Madrid (ES)**
  • **Asociación Centro de Investigación en Biomateriales - CIC biomaGune**
    **20014 San Sebastián (ES)**

(72) Inventors:
  • **CEJUDO SANCHES, Janaina**
    **28049 Madrid (ES)**
  • **TROBO MASEDA, Lara**
    **28049 Madrid (ES)**
  • **ROCHA MARTÍN, Javier**
    **28049 Madrid (ES)**

  • **GUISÁN SEIJAS, Jose Manuel**
    **28049 Madrid (ES)**
  • **GARCÍA OVEJERO, Jesús**
    **28049 Madrid (ES)**
  • **MORALES HERRERO, Maria del Puerto**
    **28049 Madrid (ES)**
  • **BUSSOLARI, Francesca**
    **50009 Zaragoza (ES)**
  • **ARMENIA, Ilaria**
    **50009 Zaragoza (ES)**
  • **MARTINEZ DE LA FUENTE, Jesús**
    **50009 Zaragoza (ES)**
  • **GRAZÚ BONAVÍA, María Valeria**
    **50009 Zaragoza (ES)**
  • **LÓPEZ GALLEGO, Fernándo**
    **20014 San Sebastián (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **HYBRID COMPOSITE FOR TUNING LOCAL NANOACTUATION OVER ENZYME ACTIVITY, WITHOUT TUNING MNP ANISOTROPY**

(57)    The present invention relates to a process for the production of a system comprising providing a porous scaffold comprising functional groups capable of covalently binding MNPs to said porous scaffold; providing at least a population of magnetic nanoparticles (MNPs) comprising functional groups capable of covalently binding the porous scaffold of step a), and contacting the MNPs with the porous scaffold under suitable conditions for the immobilization of the MNPs to the scaffold by means of covalent bonds. The present invention also provides the system obtained by the process, and uses thereof, as well as a method for carrying out an enzymatic reaction.

EP 4 397 743 A1

## Description

## TECHNICAL FIELD

The present invention relates to magnetic nanoparticles and porous materials for the control of enzymatic reactions. Particularly, it refers to a process to carry enzymatic reactions on the surface of magnetic nanoparticles in a one-pot system.

## BACKGROUND ART

Enzymes have higher selectivity, specificity and efficiency than chemical catalysts. Due to their properties and their green chemistry, biocatalysts are widely used in food, textile and pharmaceutical industry. Enzymatic and multi-enzymatic reactions have also become an important tool in biotechnology industry for the production of biopolymers, drugs or biofuels. An important limitation for the implementation of multi-enzymatic reactions in the industry is the optimization of enzymes performance, the different operational temperatures of the enzymes involved in the processes and the reduction of negative interactions among them. The multi-enzymatic reactions are usually performed in a multistep reactor or by operating at a compromised temperature. However, these approaches reduce the efficiency of the systems and result critical when thermolabile enzymes, substrates, co-factors or products are involved.

A novel strategy to overcome this limitation is the use of magnetic nanoparticles (MNPs) as local nanoheaters of the enzymes localized at the nanoparticle surface. In this context, MNPs can be used for the conjugation of enzymes thanks to their high specific surface area (o surface area per gram), high surface/volume ratio, low mass transfer limitation and their unique magnetic properties. Indeed, MNPs can be manipulated by an external alternating magnetic field (AMF) and, as a consequence of Neel and Brown relaxation, they can produce heat, a phenomenon known as magnetic hyperthermia. When an AMF is applied to a colloid of MNPs, the energy of the field is dissipated on the nanoparticle surface as heat. However, the use of magnetic heating to gain control over enzyme activity is nowadays an important limitation for the use of these systems since the effect of the heat generated by high frequencies of AMF on enzymes directly attached to MNPs has been though scarcely studied. Thus, a fine tuning of such magnetic activation is required among the suitable MNPs.

Although, AMF-tunability was observed with enzyme/MNPs hybrids, aggregation issues that could affect their magnetic heating efficiency has been observed. Indeed, in some enzyme/MNPs combinations aggregation have been observed during their functionalization, the application of AMF and/or even due to changes in pH/ionic strength of the medium.

Thus, new systems that allow a better control AMF-tunability with enzyme/MNPs hybrids are needed.

## BRIEF DESCRIPTION OF DRAWINGS

**Figure 1.** Extra level of tuning of local magnetic heating triggered by AMF due to differential spatial distribution of the MNPs within porous microbeads.

**Figure 2.** Workflow optimized to obtain the integration of MNPs and enzyme to be nanoactuated within porous microbeads.

**Figure 3.** Incubation conditions optimized to achieve the kinetic control over aminated-MNPs covalent binding on glyoxyl-agarose microbeads. Conditions used: 1 mgFe/0.5 g glyoxil agarose activated with 52 $\mu$mol of aldehyde/mL in a) sodium bicarbonate 0.1 M pH 8.0 containing 2 mM EDA at 25 °C for fast immobilization rate; and b) sodium bicarbonate 0.1 M pH 8.5 containing 0.1% CTAB and 0.05 M ethylenediamine at 4 °C for slow immobilization rate.

**Figure 4.** Binding kinetics of two different batches of MNPs using the same incubation conditions: 1 mgFe/0.5 g glyoxyl agarose activated with 52 $\mu$mol of aldehyde/mL in 0.1 M sodium bicarbonate buffer, 0.1% CTAB, 50 mM ethylenediamine pH 8.0 (final volume 5 mL), 4 °C.

**Figure 5.** Re-optimized immobilization conditions to achieve fast and slow immobilization using a batch of aminated-MNPs with a different amine content than CSIC10g batch.

**Figure 6.** Critical parameters that have been shown to affect the rate of immobilization of MNPs and therefore their spatial distribution on agarose microbeads. GFP=green fluorescent protein covalently attached to the surface of MNPs previously integrated within agarose microbeads.

**Figure 7.** SEM analysis of ultrathin slices of a hybrid embedded in methyl-cellulose resin with an expected heterogenous MNP distribution due to their fast immobilization to the agarose microbeads

**Figure 8.** Scheme representing for SAR measurements are obtained from ferrofluids and mHs samples.

**Figure 9.** SEM (SEM Inspect) images of heterogenous TLL@mHs after 5, 20, 30 min of AMF application (772kHz, 30mT). Observation conditions: dry samples, palladium coating, 10.00 kV.

**Figure 10.** SEM (SEM Inspect) images of homogenous TLL@mHs after 5, 20, 30 min of AMF application (772kHz, 30mT). Observation conditions: dry samples, palladium coating, 10.00 kV.

**Figure 11.** Global heating capacity of homogenous vs heterogenous mHs when different AMF applied conditions have been applied. As a control the global heating achieved with bare MNPs at the same Fe concentration was also determined.

**Figure 12.** Activity assay used to determine TLL lipase activity consisting of the hydrolysis pf p-NPB and the detection of the production of 4-nitrophenolate ion by the increase in absorbance at 415 nm [Sensors 2015, 15, 2798-281. Activity assay conditions: 0.4 mM p-nitrophenyl butyrate, 25 mM Phosphate pH 7.0

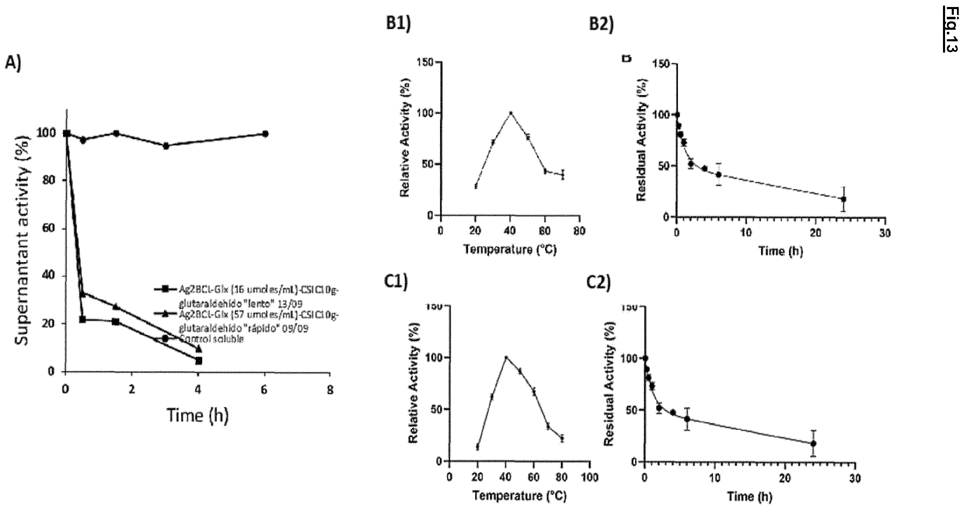

**Figure 13.** Obtention and thermal characterization of TLL homogenous and heterogenous mHs. A) Kinetics of immobilization of 3 mg/mL of TLL after overnight activation of MNPs amino groups with glutaraldehyde. (A) Fast hybrid: Ag2BCL-Glx (57 $\mu$mol CHO/mL)-CSIC10g-glutaraldehyde-TLL; (■) Slow hybrid: Ag2BCL-Glx (16 $\mu$mol CHO/mL)-CSIC10g-glutaraldehyde-TLL; (●) Soluble TLL (control). B) Agarose@CSIC10eFAST@TLL (heterogenous) hybrids characterization: B1) TLL mHs optimal temperature profile; B2) kinetic enzyme stability at 45 °C. C) Agarose@CSIC10eSLOW@TLL (homogenoeus) hybrids characterization: C1) TLL mHs optimal t profile; C2) kinetic enzyme stability at 45°C.Activity assay conditions: 10 mg of mH is mixed with 0,4 mM of p-nitrophenyl butyrate (p-NPB) in 25 mM phosphate buffer pH 7.0 (final volume 2 mL).

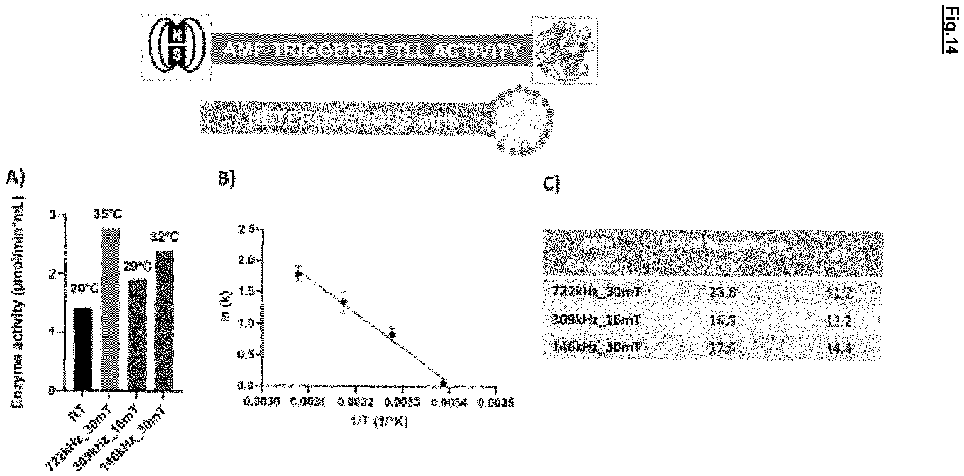

**Figure 14.** Activity of heterogenous TLL mHs hybrids achieved when applying AMF with different combinations of amplitude and frequency compared with the activity achieved using the same assay conditions but without AMF application (RT, performed at room temperature= 20°C). A) Enzyme activity reached including the hypothetical local temperature achieved for each incubation condition extrapolated from the B) Arrhenius plot of each mHs. C) Difference between the local temperature and the global temperature reached for each incubation condition. Each assay was repeated two times (n=2). Activity assay conditions: 10 mg of mH is mixed with 0.4 mM of p-nitrophenyl butyrate (p-NPB) in 25 mM phosphate buffer pH 7.0 (final volume 2 mL).

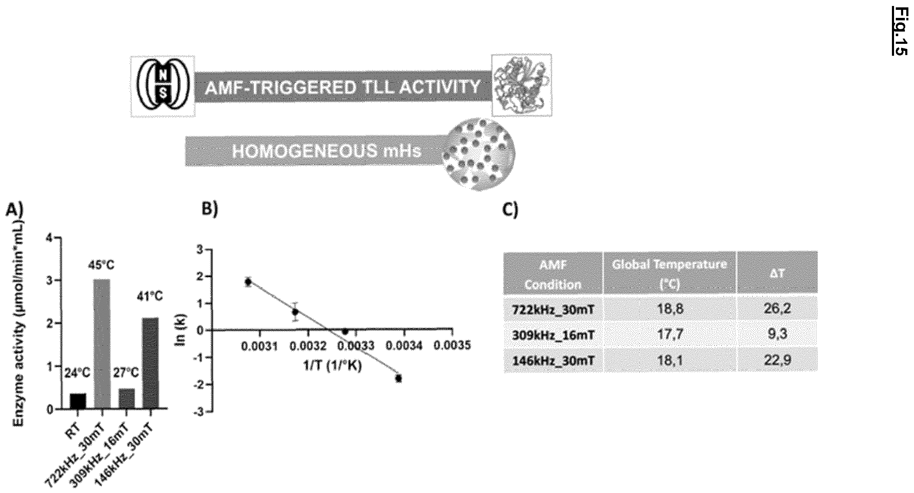

**Figure 15.** Activity of homogenous TLL mHs hybrids achieved when applying AMF with different combinations of amplitude and frequency compared with the activity achieved using the same assay conditions but without AMF application (RT, performed at room temperature= 20°C). A) Enzyme activity reached including the hypothetical local temperature achieved for each incubation condition extrapolated from the B) Arrhenius plot of each mHs. C) Difference between the local temperature and the global temperature reached for each incubation condition. Each assay was repeated two times (n=2). Activity assay conditions: 10 mg of mH is mixed with 0.4 mM of p-nitrophenyl butyrate (p-NPB) in 25 mM phosphate buffer pH 7.0 (final volume 2 mL).

**Figure 16.** Enzyme stability of heterogenous TLL@mHs while 30 minutes of: A) AMF application using 772 kHz and 30 mT; B) incubation at 45 °C using a thermoblock as global heater source. Each assay was repeated two times (n=2). Activity assay conditions: 10 mg of mH is mixed with 0.4 mM of p-nitrophenyl butyrate (p-NPB) in 25 mM phosphate buffer pH 7.0 (final volume 2 mL).

**Figure 17.** Enzyme stability of homogenous TLL@mHs while 30 minutes of: A) AMF application using 772 kHz and 30 mT; B) incubation at 45°C using a thermoblock as global heater source. Each assay was repeated two times (n=2). Activity assay conditions: 10 mg of mH is mixed with 0.4 mM of p-nitrophenyl butyrate (p-NPB) in 25 mM phosphate buffer pH 7.0 (final volume 2 mL).

**Figure 18.** (A) Chemical structure of the cofactor NAD+; (B) reduction of $NAD^+$ to NADH during enzymatic catalysis; (C) Spectral scans of NADH (blue dots) and $NAD^+$ solutions (red dots). [Adapted from https://www.biotek.com/resources/application-notes/monitoring-protein-dehydrogenase-activity-with-filter-basedabsorbance-spectroscopy/].

**Figure 19.** (A) Kinetics of immobilization of CSIC10g on glyoxil-agarose microbeads using a slow immobilization protocol (1mgFe/0.5 g glyoxil-agarose with 52 $\mu$mol of aldehyde/mL in 0.1 M sodium bicarbonate buffer, 0.1% CTAB, 50 mM ethylenediamine pH 8.0, 4 °C). (B) Kinetics of immobilization of 0.4 mg/mL TtADH after overnight activation of MNPs amino groups with glutaraldehyde.

**Figure 20.** Agarose@CSIC10i_Homogenous@TtADH hybrids characterization: (A) immobilized TtADH optimal temperature profile, (B) kinetic enzyme stability at 70 °C. Assays repeated 2 times (n=2). Activity assay conditions: 100 mg of mH is mixed with a master mix composed by: 0.1 mM of NADH, 5% 2-Propanol and 50 mM of ethyl acetoacetate (EAA) in 0.1 M phosphate buffer pH 7.0 (final volume 2 mL).

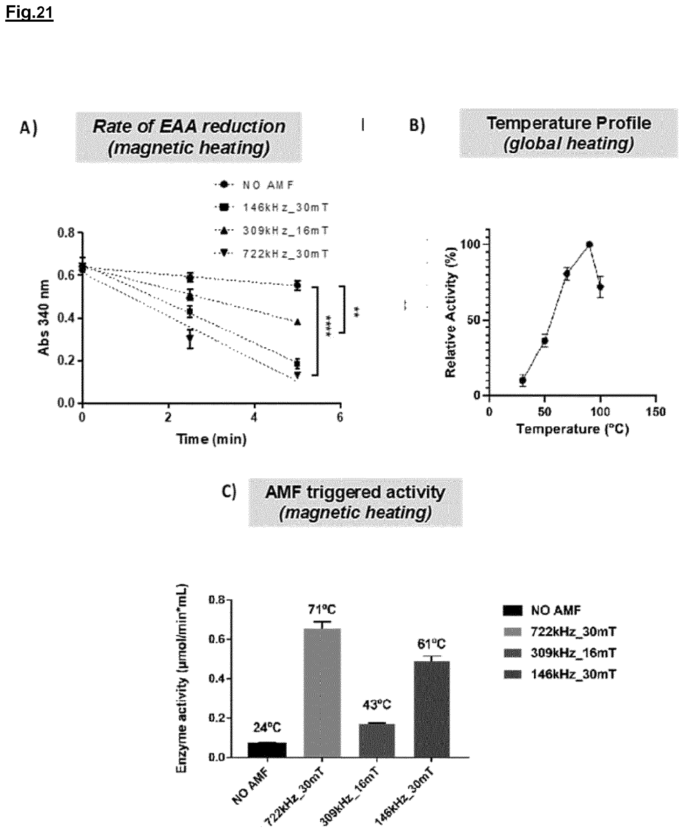

**Figure 21.** AMF-tunability of the obtained homogeneous (slow) TtADH mHs. a) Changes in the initial rate of EAA oxidation caused by the application of AMF. Initial rates were calculated from the slopes obtained when different AMF settings (frequency and field intensity) were applied and compared to the rate obtained when AMF was not applied (No AMF). b, Arrhenius plot of the different mHs used to correlate the activity increases observed with temperatures sensed by the enzymes in the microenvironment. c, AMF-triggered increase on enzyme activity observed applying different AMFs compared to the control of enzyme activity at room temperature (No AMF control). Assays repeated 3 times (n=3).

**Figure 22.** Enzyme stability of homogeneous TtADH@mHs while 30 minutes of: A) AMF application using 772 kHz and 30 mT; B) incubation at 45°C using a thermoblock as global heater source. Each assay was repeated two times (n=2). Activity assay conditions: 100 mg of mH is mixed with a master mix composed by: 0.1 mM of NADH, 5% 2-Propanol and 50 mM of ethyl acetoacetate (EAA) in 0.1 M phosphate buffer pH 7.0 (final volume 2 mL).

**Figure 23.** A) Degradation of starch by $\alpha$-amylase from *Bacillus licheniformis* (BIAm). B) Colorimetric assay used to follow the starch degradation performed by the BLAm homogenous mHs optimized. C) Temperature profile of BIAm mHs obtained using a thermoblock as global heating source.

**Figure 24.** AMF-tunability of the obtained homogenous (slow) BIAm mHs. AMF was applied during 15 min and enzyme activity compared to the activity registered at 20 °C and 90 °C (NON-AMF controls). Assays repeated 3 times (n=3).

**Figure 25.** Different coatings using the same inorganic core renders MNPs with amine groups linked via shorter or longer spacers to the MNP surface.

**Figure 26.** Main physicochemical properties of CSIC10 and CSIC13 MNPs.

**Figure 27.** Immobilization kinetic of aminated MNPs with short (CSIC10) or long (CSIC13) spacers but the same inorganic core. The protocol used for the immobilization is the protocol optimized for a slow immobilization of CSIC10g: 1 mg Fe/0.5 g Agarose 0.1 M Sodium bicarbonate buffer, 0.1% CTAB, 50 mM Ethylenediamine pH 8.0, 4 °C.

**Figure 28.** AMF-activation of TtADH attached to homogenous mHs obtained by integration of CSIC10 (short spacers) or CSIC13 (long spacers) MNPs. Activity assay conditions: 100 mg of mH is mixed with a master mix composed by: 0.1 mM of NADH, 5% 2-Propanol and 50 mM of ethyl acetoacetate (EAA) in 0.1 M phosphate buffer pH7.0 (final volume 2 mL).

**Figure 29.** TtADH@CSIC13 mHs enzyme activity increase with respect to the activity when the AMF was not applied (NON-AMF) when applying different AMF conditions Activity assay conditions: 20 mg of mH is mixed with a 2 mL master mix composed by: 0.1 mM of NADH, 5% 2-Propanol and 50 mM of ethyl acetoacetate (EAA) in 0.1 M phosphate buffer pH 7.0 (final volume 2 mL). mHs hybrids were prepared using agarose containing 15 $\mu$mol/mL. Each assay was repeated two times (n=2). The AMF was applied for 5 minutes when applied while conducting the enzyme activity assay.

**Figure 30.** Workflow proposed for the bienzymatic@MNPs microhybrids preparation.

**Figure 31** Influence of AMF application on the activity of FDH@mHs prepared with CSIC10 or CSIC13 MNPs. mHs non exposed to AMF were used as controls. FDH activity assay: 0.1 M formic acid and 0.1 M NAD⁺ in 50 mM sodium phosphate pH 7 conducted in the presence or absence of an AMF. Each assay was repeated two times (n=2). Global temperature of the reaction media was of 20 ± 0.4 °C while AMF was applied for 5 minutes.

**Figure 32.** Immobilization workflow developed for the introduction of a His-tag chelator on the surface of the MNPs previously integrated within the agarose microbeads to be able to functionalize them with recombinant proteins containing an His-tag or His-clusters.

**Figure 33.** Conjugation yield of GFP in absence (black) or in presence (pink) of 0.5 M imidazole, and elution yield of GFP after 1h incubation with 0.5 M imidazole. Assays repeated 2 times (n=2).

## GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of" does not exclude materials or steps that do not materially affect the basic and novel characteristics of the claim.

By "nanoparticle" is referred as a particle of matter that is between 1 and 100 nanometres (nm) in diameter.

By "magnetic anisotropy" (K) is meant herein the property that confers a preferred direction on the spins of a system, which may not be aligned with an external magnetic field. This definition is further developed below.

"Superparamagnetism" refers to materials that do not exhibit magnetic remanence when no magnetic field is applied but behave similar to a ferromagnetic material upon the application of a magnetic field.

By "Optimum temperature" (OT) of an enzyme is referred to the temperature range in which a maximal rate of reaction is achieved.

## DESCRIPTION OF THE EMBODIMENTS

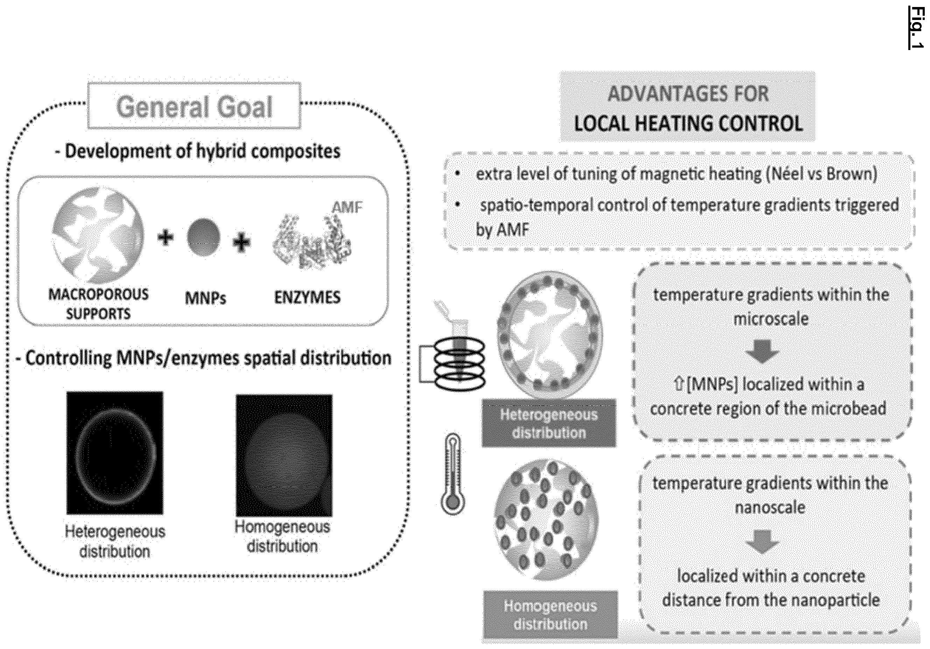

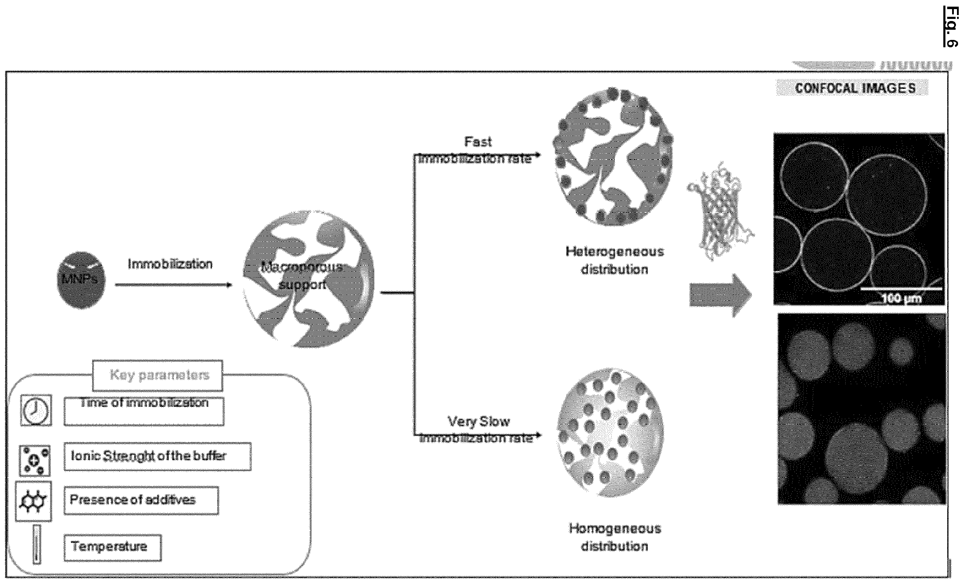

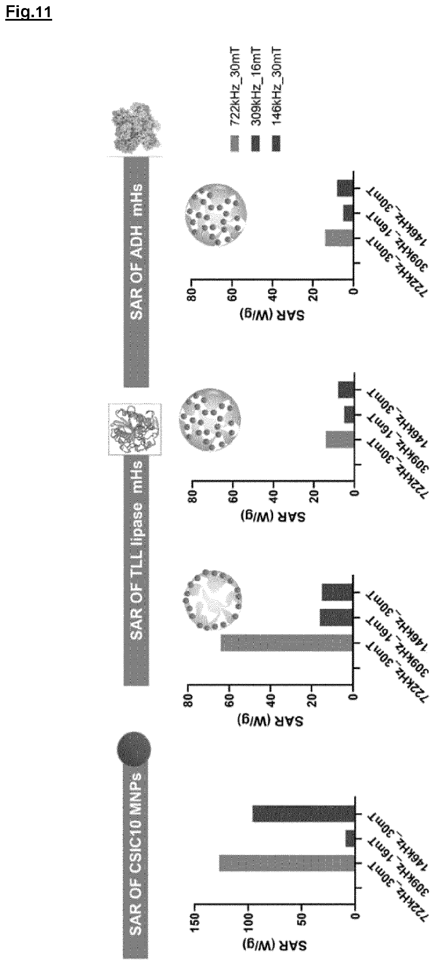

The present invention shows that it is possible to tune the magnetic heating of MNPs integrated into a scaffold formed by agarose microbeads. This is possible due to the methodology used to immobilize the MNPs to the scaffold. Two immobilization methods were tested herein, named homologous and heterologous immobilization. Homologous immobilization is characterized by being a slow immobilization, while heterologous immobilization is a fast integration of the MNPs to the scaffold. As a result, the homologous immobilization provides a system of MNPs integrated in a scaffold in a regular and evenly form where the MNPs are magnetically isolated from each other, while the heterologous immobilization provides a system where there is a high concentration of MNPs attached to the surface or outer layer of the scaffold (see Fig 6). The difference in the MNPs distribution affects the efficiency of the heating of the MNPs triggered by AMF application (Figure 1), showing that spatial location is an additional mechanism for tuning local temperature gradient achieved at the MNPs environment. This is demonstrated herein with the functionalization of the MNPs with enzymes, where it could be observed although using systems having the same total amount of Fe, heterogenous systems has a clear higher magnetic heating capacity when high AMF conditions are applied (see Figs. 11-15). From the results obtained, it can be concluded that the local heating of an enzyme attached to MNPs that were previously covalently integrated within microporous agarose beads is feasible. Besides, it is also possible to trigger high local temperatures like the optimal temperature of the thermophilic enzymes.

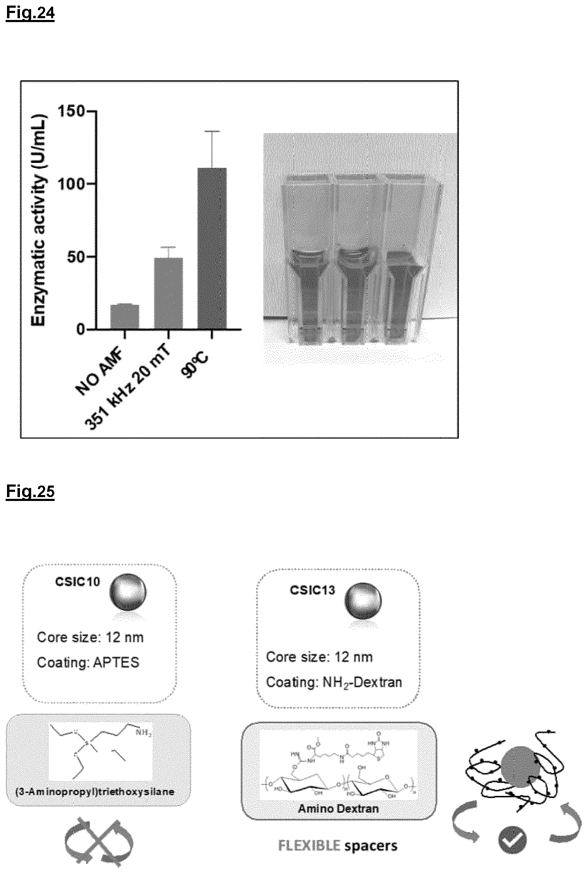

A further way of tuning the local heating on the surface of MNPs is to coat them with spacers that separate the enzymes functionalized on the MNPs from the core of the MNP. The longer the spacer is, the more mobility the MNP has, achieving higher local temperatures. On the contrary, shorter spacers limit the mobility and thus the heating of the MNPs is more limited. This way, the rotation capacity of the integrated MNPs is controlled, thereby controlling their heating capacity, as shown in Figs 25-31. Thus, different complementary assays have been conducted that support that the way MNPs are fixed to the agarose fibres reducing or promoting their AMF-triggered rotation (trough multipoint short bonds or flexible spacers) could also be a way of tuning the magnetic heating efficiency of the MNPs. This additional strategy could be used in combination with other strategies that we have showed that also allow tuning their magnetic heating efficiency as it is the control of their distribution within the agarose microbeads and/or tunning the AMF conditions applied.

In a **first aspect,** the present invention provides a process or method for the production of a system comprising a scaffold and magnetic nanoparticles (MNPs), the process comprising the steps of:

  a) providing a porous scaffold,
  b) providing at least a population of MNPs, characterized in that the MNPs have a magnetic anisotropy capable of dissipating local thermal energy on their surface upon application of an external alternating magnetic field (AMF), and
  c) contacting the MNPs with the porous scaffold under suitable conditions for the immobilization of the MNPs to the porous scaffold.

Preferably, the first aspect provides a process or method for the production of a system comprising a scaffold and magnetic nanoparticles, the process comprising the steps of:

a) providing a porous scaffold, wherein the porous scaffold comprises or is activated and/or coated to provide on its surface hydroxyl, carboxyl, carbonyl, amino, thiol, azide, or alkyne functional groups capable of covalently binding MNPs to said porous scaffold;

b) providing at least a population of magnetic nanoparticles (MNPs), characterized in that the MNPs have a magnetic anisotropy capable of dissipating local thermal energy on their surface upon application of an external alternating magnetic field (AMF), wherein the MNPs comprise or are coated and/or activated to provide on their surface hydroxyl, carboxyl, carbonyl, amino, thiol, azide, or alkyne functional groups capable of covalently binding to the porous scaffold of step a), and

c) contacting the MNPs with the porous scaffold under suitable conditions for the immobilization of the MNPs to the scaffold by means of covalent bonds,

wherein steps a) and b) can be performed in any order.

By "coating" is meant the modification of the surface of the MNPs or the scaffold with the application of at least one layer of a material (also called a shell) usually giving the surface of the scaffold or MNP new properties. The coating may be mechanical, physical, chemical and/or biological. One or more coating layers may be added to the MNPS and/or to the scaffold. Biological coatings are also referred herein as functionalization with a biomolecule.

By "activating the scaffold" or "activating the MNPs" is referred herein as the use of reagents to 'activate' groups to bind molecules/biomolecules, preferably covalently. "Activation" is needed when the groups at the scaffold or MNPs exhibit low reactivity in an aqueous environment (e.g. carbodiimide for binding to COOH groups), or in order to join groups that are simply not reactive toward one another (e.g. $NH_2$ to $NH_2$).

In a preferred embodiment, the process is carried out in a one-pot reaction medium.

Each of the steps of the first aspect (steps a), b), c) and the optional step of d) are explained in detail below. It is noted that all the embodiments comprised in each of steps a), b), or c), and the optional step d), can be combined among them and with any other embodiments comprised in any other different step. It is thus understood that any possible combination between embodiments from the same and from different step is also encompassed in the present invention.

Step a) of the first aspect comprises providing a porous scaffold. "Porous scaffold" as referred herein means a material having external pores on the surface and/or internal pores in the core. Preferably, the pores are interconnective. According to their pore sizes, the scaffolds can be classified as: microporous materials (less than 2 nm), mesoporous materials (2-50 nm), macroporous materials (50-200 nm), and gigaporous materials (more than 200 nm).

In an embodiment, the porous scaffold comprises beads and/or is a hydrogel. In an embodiment, the porous scaffolds are mesoporous hydrogels/beads (with size of porous of 2-50 nm), macroporous hidrogels/beads (with pore size of 50-200 nm), or hydrogels/gigaporous beads (with pore size of more than 200 nm). Preferably, the pore size of the hydrogel or the beads is more than 50nm (microporous and gigaporous scaffolds), preferably between 50-200nm (microporous scaffolds). Pore size can be determined by the skilled person using known methods, such as by scanning electron microscopy (SEM), transmission electron microscopy (TEM), or confocal laser scanning microscopy (CLSM).

Preferably, the scaffold comprises porous beads, wherein preferably the porous beads have a spherical substantially spherical geometry. Preferably, the beads have a size of 10-300 $\mu$m, preferably 50-150 $\mu$m or 50-200 $\mu$m. In a preferred embodiment, the porous scaffold preferably comprises cross-linked agarose beads. More preferably, the porous scaffold comprises BCL-crosslinked agarose beads having spherical geometry with a size of 50-150 $\mu$m (bead mean diameter of 90 $\mu$m) and exclusion limit of $40 \times 10^6$ Da.

Materials and methods used to obtain porous scaffolds with the desired range of porosity are known in the art. They can also be obtained commercially. In an embodiment, the materials used are selected from polymers or co-polymers [e.g. polylactic acid; poly(lactic-co-glycolic acid); chitosan, polycaprolactone; poly(divinylbenzene)]; ethylene glycol dimethacrylate; methacrylamide; N,N'-methylen-bis(acrylamide); oxirane acrylic polymers, polystyrene sulfonate), organic materials (e.g. agarose, cross-linked agarose, K-carrageenan, cellulose, starch, collagen, chitosan), inorganic materials (mesoporous/macroporous silica, calcium carbonate, hydroxyapatite, porous glass, zirconia, titania), and metal-organic frameworks (MOF).

In an embodiment, the porous scaffold:

- comprises hydroxyl, carboxyl, carbonyl, amino, thiol, azide, or alkyne functional groups capable of covalently binding MNPs and optionally biomolecules, such as enzymes, to said porous scaffold, and/or
- is coated with hydroxyl, carboxyl, carbonyl, amino, thiol, azide, or alkyne functional groups capable of covalently binding MNPs and optionally biomolecules, such as enzymes, to said porous scaffold, and/or
- is activated to provide hydroxyl, carboxyl, carbonyl, amino, thiol, azide, or alkyne functional groups capable of covalently binding MNPs and optionally biomolecules, such as enzymes, to said porous scaffold.

In an embodiment, the porous scaffold comprises microbeads, preferable agarose microbeads. In an embodiment, the functional groups comprised in the porous microbeads are carbonyl groups, and are more preferably aldehyde groups. In this sense, aldehyde agarose microbeads are especially preferred as these allow the covalent binding of agarose to the amino groups of any chosen biomolecule (peptides, enzymes, antibodies and etc.). Preferably, the bond between the aldehyde agarose microbeads and the biomolecule is irreversible. Aldehyde agarose microbeads can be obtained by activation or coating as known by the skilled person and can also be found in the market as ready to use and require no activation. As regards the activation of agarose to provide aldehyde agarose microbeads, it is noted that oxidation of glycols (or glyceryl groups) with Na-periodate is a stoichiometric reaction. Therefore, the activation degree of agarose microbeads can be easily controlled through the periodate concentration used (this method depends on the oxidation of cis-vicinal hydroxyl groups of agarose by sodium metaperiodate (NaIO4) to generate aldehyde functions). In particular, Pe, for obtaining 10 wet grams of glyoxyl-activated agarose with an activation degree of 50 $\mu$moles of aldehyde groups/mL of agarose (71,43 $\mu$moles of aldehyde groups/wet gram of agarose), 7.14 mL of oxidation solution (0.1 M Na-periodate) have to be added to oxidaze agarose.

In any case, the coupling reaction of aldehyde agarose microbeads to the amino groups of any chosen biomolecule involves spontaneous formation of Schiff base bonds between aldehydes groups (on the scaffold) and amines (on the ligand, i.e., the biomolecule). In a preferred embodiment, the Schiff base bonds are subsequent stabilized by incubation with a reductant (such as sodium cyanoborohydride or sodium borohydride). The entire coupling reaction occurs in about 2 to 6 hours in simple non-amine buffers such as PBS or borate.

Therefore, in an embodiment, the porous scaffold comprises aldehyde agarose microbeads, wherein the concentration of the aldehyde groups is at a range of about 100 $\mu$moles, preferably between 1-80, 1-70, 1-60 $\mu$moles of aldehyde groups/wet gram of agarose microbeads. In a preferred embodiment, the concentration of the aldehyde groups is in the range of 4-60 $\mu$moles of aldehyde groups/wet gram of agarose microbeads.

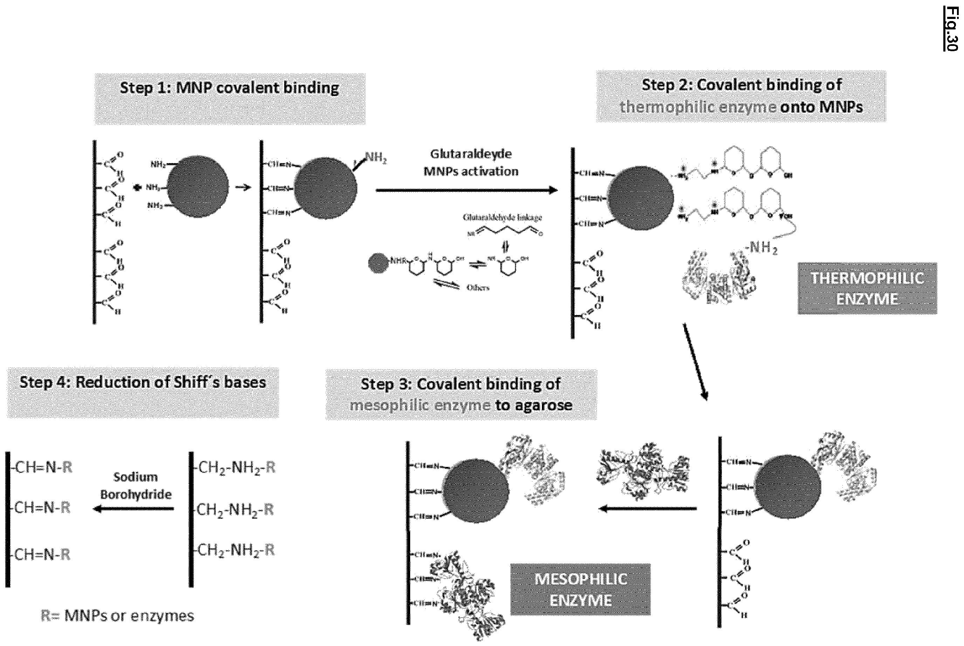

The functional groups provided on the scaffold also allow the functionalization of the scaffold with a biomolecule. Thus, in an embodiment, the porous scaffold, preferably the porous agarose microbeads, may be functionalized with at least one biomolecule, such as a peptide, protein, enzyme, antibody, or nucleic acid. This may be advantageous if a system comprising the scaffold and MNPs wants to be formed wherein each have different enzymes that work at different temperatures, as shown in Fig. 30. By "functionalization" is referred herein to as a type of coating that involves the immobilization or conjugation with a biological molecule.

Preferably, the porous scaffold comprises microbeads, preferable agarose microbeads, wherein the microbeads comprise or are coated and/or activated to provide hydroxyl, carboxyl, carbonyl, amino, thiol, azide, or alkyne functional groups to covalently integrate MNPs and optionally at least one biomolecule. Preferably, the scaffold comprises microbeads, preferable agarose microbeads of a size of between 50-150 $\mu$m with a pore size of between 50-200 nm, wherein the microbeads comprise or are preferably coated and/or activated to provide on their surface hydroxyl, carboxyl, carbonyl, amino, thiol, azide, or alkyne microbeads to covalently integrate MNPs and optionally at least one biomolecule. Preferably, the biomolecule functionalized on the porous microbeads is at least one enzyme, preferably selected from the group consisting of thermophilic, hyperthermophilic, or most preferably mesophilic enzymes. By "thermophilic" enzymes is meant enzymes that work optimally between 50 and 80 °C. By "hyperthermophilic" enzymes are meant enzymes that work optimally at more than 80 °C. By "mesophilic" enzymes are meant enzymes that work optimally at around 37 °C.

In an embodiment, the functional groups of the scaffold are activated before step c).

Step b) of the first aspect comprises providing a population of magnetic nanoparticles. "Magnetic nanoparticles" (MNPs) as referred herein are a class of nanoparticles that can be manipulated using magnetic fields. By "population of MNPs" is meant a plurality MNPs that are homogeneous in their sizes and shapes, and that form a colloidal dispersion. By "homogeneous" is meant that the particles in each of the populations of MNPs according to the first aspect are characterized by having substantially the same particle size or diameter, that is, for being substantially monodisperse. As used herein, "monodisperse" refers to MNPs possessing a narrow average particle size distribution (PSD). By "particle size distribution" (PSD) is meant herein an index (means of expression) indicating what sizes (particle size) of particles are present in what proportions (relative particle amount as a percentage where the total amount of particles is 100 %) in the sample particle group to be measured. Preferably, particle size distribution can be measured from a statistically valid analysis of hundreds of particles from TEM images, and it refers to the standard deviation of the fitting. By "colloidal" is meant that the plurality of MNPs of the populations of MNPs remain suspended in solution at equilibrium, that is, they do not tend to aggregate or flocculate.

In an embodiment, the plurality of MNPs are at least 70%, 80%, 90%, 95%, 96%, 97%, 98%, 99%, or 100% identical among them in a population of MNPs. By "identical" is meant that the MNPs are substantially similar in their characteristics, including shape, composition, magnetic properties, coating and functionalization. Preferably, the MNPs of the populations of MNPs according to the first aspect of the present invention or any of its embodiments are at least 85%, 90%, 92%, 95%, 98%, 100% identical among them, more preferably 95% identical.

In an embodiment, the population of MNPs have a particle size distribution of less than 0.5, 0.4, 0.3, 0.25, 0.2, preferably less than 0.1, or 0.05. The particle size and PSD of nanoparticles can be determined using numerous commercially available instruments and methods known by the skilled artisan. An example of this method is photon correlation spectroscopy (PCS) that the dynamic fluctuation of the scattered light intensity is the basis for calculation of the average particle size.

In an embodiment, each MNP comprises a magnetic core and an inorganic or organic coating outer shell, in which is the outer shell that plays a threefold role: it protects the core from dissolution, enhances magnetic properties of the nanoparticle by keeping them apart and introduces an anchoring point for enzyme conjugation. Preferably, the coating is an organic shell and the core is magnetic.

The core of the MNPs may be made of different inorganic materials. In an embodiment the core of MNPs is made of pure inorganic materials, such as Fe, Co or Ni; metal oxides such as iron oxide, or metal alloys such as FePt and CoPt. In an embodiment, the core of the MNPs can include Fe, Co, Ni, FePt, or SmCo. More preferably, the core of the MNPs is a ferromagnetic core selected from the group consisting of iron oxide, magnetite or maghemite. In some embodiments, the iron oxide core includes a dopant material.

The dopant material can include a metal selected from the group consisting of Mn, Co, Ni, Zn, and ZnMn.

In a preferred embodiment, the MNPs are characterized by having a particle size distribution of less than 0.1, and have a core selected from the group consisting of iron oxide, magnetite or maghemite.

In an embodiment, the average particle diameter of the core of the MNP ranges from 1 to 100 nm. In another embodiment, the average particle diameter of the MNP ranges from 5 and 50 nm. Preferably, the average particle diameter of less than 200 nm, preferably between 8 and 50 nm. More preferably, the average particle diameter of the MNPs may be less than or equal to about 50 nm, 40 nm, 30 nm, 20 nm, 10 nm or 5 nm. In another embodiment, the average particle diameter ranges from 5-50 nm, 5-40 nm, 5-30 nm, 5-20 nm, 8-15 nm, 18-25 nm, 25-35 nm or any value within these ranges. In another embodiment, the average particle diameter of the MNP is at least 5 nm, 6 nm, 7 nm, 8 nm, 9 nm, 10 nm, 11 nm, 12 nm, 13 nm, 14 nm, 15 nm, 16 nm, 17 nm, 18 nm, 19 nm, 20 nm, 21 nm, 22 nm, 23 nm, 24 nm, 25 nm, 26 nm, 27 nm, 28 nm, 29 nm, 30 nm, 31 nm, 32 nm, 33 nm, 34 nm or 35 nm.

In an embodiment, the population of MNPs can have different shapes, such as spherical, cubic, prism, rod, tubes, cylindrical, squared, etc. Preferably, the MNPs are spherical or substantially spherical.

In an embodiment, the population of MNPs are in a concentration within the range of 0.0001-10 mg of Fe/wet g of scaffold. In a further embodiment, MNPs are in a concentration within 0.0001-4 mg of Fe/wet g of scaffold. In further embodiment, MNPs are in a concentration within 0.0001-1 mg of Fe/wet g of scaffold. Preferably, MNPs are in a concentration equal or greater than 1.6 mg of Fe/wet g of scaffold.

Methods to synthesize magnetic nanoparticles of the desired size are known in the art. In an embodiment, the methods for generating the MNPs are selected from precipitation, microemulsion, thermal decomposition, solvothermal, sonochemical, microwave and assisted, chemical vapor deposition, combustion, carbon arc, laser pyrolysis, electrochemical synthesis, microorganism or bacterial synthesis. In a preferred embodiment, the method used herein to synthetize the MNPs is the coprecipitation in aqueous media.

Further, in an embodiment, the populations of MNPs have the same magnetic anisotropy. The magnetic anisotropy "K" is meant herein the property that confers a preferred direction on the spins of a system, which may not be aligned with an external magnetic field. In the base of this parameter the anisotropy field "HA" is defined as:

$$HA = 2K/MS$$

being K the anisotropy constant of the system and MS the saturation magnetization, intrinsic properties of the material depending on the chemical and crystalline nature, the particle size and shape, and aggregation state. The HA is commonly used to define the limit at which the MNPs dissipate heat or not, and it is of primary importance in determining the magnetic properties of particle systems, because it weights the relative importance of the Zeeman energy.

In an embodiment, the HA of the population of MNPs is selected from the group consisting of HA of about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 mT. In a preferred embodiment, the HA of the population of MNPs is selected from the group consisting of HA of 27 mT ± 25%, 34 mT ± 25%, or 41 mT ± 25%. In a further preferred embodiment, the HA of the population of MNPs consists of HA of 27 mT.

For a randomly oriented set of MNPs, the maximum magnetic loop area is $\approx 2KV$, with K the particle anisotropy constant and V its volume. Considering this, the equation above indicates that the amount of heat dissipated can be increased by increasing the frequency, or alternatively by using MNPs with higher anisotropy. But this is only true if the intensity of the applied field is high enough to overcome the particle anisotropy. Otherwise, the MNPs with high anisotropy only reproduce minor magnetic loops and the amount of heat generated is lower than the one of MNPs with smaller K.

MNPs with excellent bulk physical and chemical properties may not possess suitable surface properties for specific applications that are conferred by the shell. For this reason, surface coating and modification techniques that can transform these materials into valuable finished products are an important part of nanomaterials. In an embodiment, the MNPs may be surface coated to improve their stability and/or to make them biocompatible for further functionalizations such as, for example, with enzymes. Thus, the coating can be performed with one or more compounds, such as with metal ions, surfactants, small molecules, alkoxysilanes, polymers, chelating agents or biomolecules such as enzymes. Preferably, the coating is performed by means of covalent bonds between the shell and the MNP core.

In an embodiment, the MNPs are coated with an inorganic material or shell. In another embodiment, the MNPs are coated with an organic material or shell. In an embodiment, the coating shell has a thickness ranging from 0.1 nm to 20 nm. In a further embodiment, the shell has a thickness ranging from 1 to 20 nm. In further embodiment, the shell has a thickness of 1, 2, 3, 4, 5, 6, 7, 8, 9, 10, 11, 12, 13, 14, 15, 16, 17, 18, 19 or 20 nm.

In a preferred embodiment, the population of MNPs:

- comprises hydroxyl, carboxyl, carbonyl, amino, thiol, azide, or alkyne functional groups capable of covalently binding the MNPs to the porous scaffold of step a) and to the biomolecules, and/or
- are coated with hydroxyl, carboxyl, carbonyl, amino, thiol, azide, or alkyne functional groups capable of covalently binding the MNPs to the porous scaffold of step a) and to the biomolecule, and/or
- are activated to provide hydroxyl, carboxyl, carbonyl, amino, thiol, azide, or alkyne functional groups capable of covalently binding the MNPs to the porous scaffold of step a) and to the biomolecule.

In an embodiment, the MNPs are characterized by comprising or being coated and/or activated to provide on their surface hydroxyl, carboxyl, carbonyl, amino, thiol, azide, or alkyne functional groups. Preferably, the MNPs are characterized by comprising or being coated and/or activated to provide on their surface amino functional groups, i.e., the MNPs are aminated. In a preferred embodiment, the MNPs are characterized by comprising or being coated and/or activated to provide on their surface (3-aminopropyl) triethoxysilane (APTES) and aminated-dextran (DEX), or any compound derived thereof, or any combination thereof. In an embodiment, the concentration of the material placed on the surface of the MNPs is of around 50% respect to the mass of their cores.

The functional groups provided on the MNPs allow the functionalization of the MNPs with a biomolecule, such as an enzyme. The term functionalization has been defined above and also applies herein. In an embodiment, the MNPs are coated with at least one biomolecule, wherein the biomolecule is preferably selected from the list consisting of proteins, peptides, enzymes, antibodies, or nucleotides. Preferably, the at least one biomolecule is at least one enzyme.

It is noted that in certain embodiments of the present specification, the biomolecules can be immobilized to the MNPs of the present invention, preferably once these MNP are immobilized on the scaffold, by any type of suitable chemistry such as conventional bioconjugation strategies or click chemistry. In this sense, the surface of the nanoparticles may be coated with a number of different functionalities, depending upon the coating material and the reactive groups present on the targeting ligand. Most commonly, amines or carboxylic acids are present on the nanoparticle surface for reaction with ligands. These groups are readily interchangeable. Reaction of amine-functionalized particles with succinic anhydride or glutaric anhydride, converts the surface to carboxylates, whereas the activation of carboxylic acid functionalized particles with 1-ethyl-3-(dimethylaminopropyl)carbodiimide (EDC) and N-hydroxysuccinimide (NHS) or N-hydroxysulfosuccinimide (sulfo-NHS) followed by reaction with a diamine results in amine-functionalized nanoparticles. Amine-coated nanoparticles are easily modified by ligands containing NHS-modified carboxylic acids or isothiocyanates, such as fluorescein isothiocyanate (FITC). Epoxides and anhydrides can also be directly reacted in sodium bicarbonate buffer at pH 8.6. In order to functionalize these particles with carboxylic acid containing ligands, the reaction must occur in the presence of activating agents such as EDC and NHS or sulfo-NHS. Ligands bearing sulfhydryl groups, such as those present in peptides, antibodies, and oligonucleotides, can also be appended to the surface of amine-coated particles once the surface is modified with heterobifunctional linkers such as succinimidyl iodoacetate (SIA), N-succinimidyl-3-(2-pyridyldithio)propionate (SPDP), or succinimidyl-4-(N-maleimidomethyl)cyclohexane-1-carboxylate (SMCC). One of the main advantages of utilizing SPDP is that it can be used to determine the number of reactive amines on the particle, as pyridine-2-thione is released upon reaction with dithiothreitol (DTT), can be detected spectrophotometrically, and thus quantitated. Reaction of ligands with SPDP functionalized particles also liberates pyridine-2-thione, thus allowing the number of conjugated species to be determined.

On the other hand, the Huisgen 1,3-dipolar cycloaddition, also known as the "click reaction," is also highly useful in the functionalization of iron oxide nanoparticles. These reactions have several advantages over the above bioconjugations. First, click reaction can occur under relatively mild conditions in aqueous solutions. These reactions are also highly specific, occur with high yields, and do not produce undesirable side products, such as dicyclohexylurea, which is resultant from the use of the activating agent dicyclohexyl-carbodimide (DCC).

In an embodiment, the MNPs are characterized by comprising or being coated and/or activated to provide hydroxyl, carboxyl, carbonyl, amino, thiol, azide, or alkyne functional groups, and with a at least one biomolecule, wherein the at least one biomolecule is bound to the functional group directly, preferably by covalent bonds. This is referred herein as "direct functionalization" of the biomolecule on the coated MNPs. In an embodiment, the means for direct functionalization of the biomolecule to the surface of the MNPs comprise amine reactive chemistries, sulfhydryl reactive chemistries, carbonyl reactive chemistries, or hydroxyl reactive chemistries.

In an embodiment, the functionalization of the at least one biomolecule is carried out by means of amine reactive groups previously present on the surface of the MNPs, wherein the amine reactive groups are preferably selected from the list consisting of cyanogen bromide, N-hydroxy succinimide esters, homobifunctional or heterobifunctional NHS-ester crosslinkers, Mannich reactions, imidoesters, reductive amination, carbodiimide-mediated bond formation, or isothiocyanate group. Preferably, the biomolecule is an enzyme, wherein said enzyme is immobilized to the MNPs by means of covalent bonds, and wherein the covalent bonds are preferably carried out by reacting an amino group present on the surface of the MNP with a carboxyl group comprised in the enzyme.

In an embodiment, the functionalization of the at least one biomolecule is carried out by means of sulfhydryl reactive groups, wherein the sulfhydryl reactive groups are preferably selected from the list consisting of maleimide, pyridyl disulfide, or epoxy activation, and wherein the sulfhydryl reactive groups are preferably comprised in the surface of the MNPs.

In an embodiment, the functionalization of the at least one biomolecule is carried out by means of carbonyl reactive groups, wherein the carbonyl reactive groups are preferably selected from the list consisting of hydrazide or reductive amination, and wherein the carbonyl reactive groups are preferably comprised in the surface of the scaffold, preferably porous microbeads.

In an embodiment, the functionalization of the at least one biomolecule is carried out by means of hydroxyl reactive groups, wherein the hydroxyl reactive groups are preferably selected from the list consisting of epoxy activation chemistries, divinylsulfone, or cyanuric chloride, cyanuric bromide, and wherein the hydroxyl reactive groups are preferably comprised in the surface of the MNPs.

The functionalization of the biomolecule to the MNPs may be an indirect functionalization, wherein the indirect functionalization is characterized by the addition of a chelating agent that acts as a bridge or ligand between the at least one biomolecule and the functional group the MNP. Thus, in an embodiment, the MNPs are characterized by comprising or being coated and/or activated to provide hydroxyl, carboxyl, carbonyl, amino, thiol, azide, or alkyne functional groups, and a at least one biomolecule, wherein the at least one biomolecule is bound to the functional group indirectly, preferably by metal affinity interactions. This is referred herein as "indirect functionalization" of the biomolecule on the coated MNPs. Chelation is a type of bonding of ions and molecules to metal ions. It involves the formation or presence of two or more separate coordinate bonds between a polydentate ligand and a single central metal atom. These ligands are called chelants, chelators, chelating agents, or sequestering agents. This type of indirect functionalization is also referred herein metal affinity based-functionalization. By "metal affinity interaction/conjugation/functionalization" is referred herein as to the reaction that occurs when metal-coordinating residues at the protein surface (eg. histidine, cysteine) specifically interact forming complexes with transition metal ions fixed to a solid scaffold.

Preferably, the at least a chelating agent comprises at least a divalent metal ion ($Me^{2+}$). The type of indirect functionalization using a chelating element as a bridge between the coated MNP and the biomolecule is also called metal affinity based-conjugation. In a preferred embodiment, the at least a divalent metal ion is selected from the group consisting of a divalent metal ion is selected from the group consisting of $Cu^{2+}$, $Co^{2+}$, or $Ni^{2+}$. In a preferred embodiment, the chelating agent comprises nitriloacetic acid derivatives (NTA), and the at least a divalent metal ion is selected from the group consisting of $Cu^{2+}$, $Co^{2+}$, or $Ni^{2+}$. In an embodiment, the concentration of the chelating agent coated on the MNPs is at a concentration range of 50 $\mu$M $\pm$ 50%. In a preferred embodiment, the concentration of the chelating agent coated in the MNPs is at a mass percentage in the range of 5-20% respect to the mass of their cores.

Preferably, the chelating agent is nitrile acetic acid. In a preferred embodiment, the concentration of the nitrile acetic acid agent in the MNPs is at a concentration range of 0.05 $\mu$mol NTA-Me / mg Fe $\pm$ 50%.

Thus, in a preferred embodiment, the MNPs are characterized by comprising or being coated and/or activated to provide on their surface hydroxyl, carboxyl, carbonyl, amino, thiol, azide, or alkyne functional groups and with a chelating agent. Preferably, the coating or activation of the hydroxyl, carboxyl, carbonyl, amino, thiol, azide, or alkyne functional groups is performed before immobilization of the MNPs to the porous scaffold, and the coating with the chelating agent is performed after immobilization of the MNPs to the porous scaffold. In a preferred embodiment, the MNPs are characterized by comprising or being coated and/or activated to provide on their surface amino functional groups and with a chelating agent before being functionalized with at least one biomolecule. The chelating agent can be advantageously introduced for the oriented binding of recombinant His-tagged enzyme variants. Thus, preferably, the biomolecule comprises a histidine tag. Preferably, the at least one biomolecule is an enzyme, wherein preferably said enzyme comprises at least one histidine-tag. Preferably, the tag is six consecutive histidines.

Therefore, in an embodiment of the first aspect, the population of MNPs is characterized in that they are first coated with at least one functional groups, preferably amino functional group, and subsequently functionalized, either directly or indirectly, with at least one biomolecule, preferably at least one enzyme. Preferably, the at least one biomolecule, preferably at least one enzyme, is the same among the plurality of MNPs forming the population. Alternatively, the at least one biomolecule, preferably at least one enzyme, is different among the plurality of MNPs forming the population.

In a preferred embodiment, the at least one enzyme functionalized on the surface of the MNPs is selected from the group consisting of thermophilic, hyperthermophilic, or mesophilic enzymes.

In an embodiment, the at least one enzyme functionalized on the surface of the MNPs is selected from the group consisting of oxidoreductases, transferases, hydrolases, lyases, ligases, and isomerases. In a preferred embodiment, the enzymes are selected from the group consisting of sucrose phosphorylase from *Leuconostoc mesenteroids, Bacillus stearothermophilus* alcohol dehydrogenase, *Thermus thermophilus* alcohol dehydrogenase, amylase from *Bacillus licheniformis*, glucose isomerase from Suncus murinus, cellobiose phosphorylase from Clostridium thermocellum, L-aspartate oxidase from *Sulfolobus tokodaii, Bacillus megaterium* or *Chromobacterium violaceum* transaminases, Acetobacter pasteurianus pyruvate decarboxylase, collagenase from *Bacillus sp,* hyaluronidase from *Thermasporomyces composti, Thermus thermophilus* lipase and NADH-oxidase, horseradish peroxidase, uracil phosphoryl transferase from *Saccharolobus shibatae,* or cytosine deaminase from *Escherichia coli.*

In an embodiment, the amino MNPs are activated before being functionalized with the at least one molecule. The activation may be performed using amine-reactive crosslinkers, which can be used for the direct or indirect functionalization of the at least one biomolecule via amine groups or via a chelating agent coated on the amino MNPs, respectively. Preferably, the amine-reactive crosslinkers are glutaraldehyde, bis(sulfosuccinimidyl)suberate, homobifunctional or heterobifunctional NHS-ester crosslinkers. Thus, in a preferent embodiment, glutaraldehyde is used for the direct covalent functionalization of the biomolecule, preferably enzyme, to the surface of the MNP via condensation of amines at the MNPs surface by Mannich reactions and/or reductive amination. In another embodiment glutaraldehyde is used to anchor chelating agents, preferably nitrile acetic acid, to the MNP coating for site-specific metal affinity based-functionalization of his-tagged recombinant enzymes by the use of immobilized metal ions, preferably cobalt, nickel, copper, zinc.

In an embodiment, the MNPs are coated with APTES and DEX, since it provides colloidal stability at a wide pH range and the possibility to anchor the MNPs to the porous scaffold and the subsequent direct binding of enzymes or chelating agents in order to bind his-tagged recombinant proteins by metal affinity binding.

Depending on the material used in the coating of the MNP, the at least one biomolecule functionalized on said MNP may be closer or further to the MNP surface. The distance of the biomolecule to the surface of the MNP is relevant for the control of the heat that the biomolecule receives from the MNPs upon application of an AMF, as will be explained below. In an embodiment, the at least one biomolecule functionalized on the surface of the MNPs is separated from the surface of the MNPs by a distance of more than 1 nm. This distance is achieved by coating the MNP with a linker that provides functional groups separated from the surface of the MNPs by a distance of more than 1 nm. The separation of the functional group, by a distance of more than 1 nm from the MNPs provides more rotation to the biomolecule bound thereto, achieving higher temperatures upon application of an AMF, as will be explained below. Thus, in a preferred embodiment, the MNPs comprises or is coated and/or activated with functional groups present on the surface of the MNPs and capable of binding to the biomolecule, wherein said functional groups are separated from the surface of the MNPs by a distance of more than 1 nm.

In an embodiment, the at least one biomolecule functionalized on the surface of the MNPs is separated from the surface of the MNPs by a distance of equal or less than 1 nm. This separation is achieved by coating the MNP with a linker that provides functional groups separated from the surface of the MNPs by a distance of equal or less than 1 nm. The separation of the functional group of the MNP by a distance of equal or less than 1 nm from the MNPs provides a more restricted rotation to the biomolecule bound thereto, achieving lower temperatures upon application of an AMF, as will be explained below. Thus, in a preferred embodiment, the MNPs comprises or is coated and/or activated with functional groups present on the surface of the MNPs and capable of binding to the biomolecule, wherein said functional groups are separated from the surface of the MNPs by a distance of equal or less than 1 nm.

Preferably, the separation of the functional groups to the MNPs' surface is provided by a linker molecule. By "Linker molecule" is referred herein to a chemical substance capable of reacting with both the polymer and the functional molecules. Linkers molecules are used when functional molecules cannot be directly grafted onto the polymer surface or when the biomolecule wants to be functionalized at a certain distance of the MNP or scaffold. In a preferred embodiment, the linker is selected from the group consisting of amino dextran, aminated starch, polyethylenimine, polylysine, bifunctional derivatives of polyethylene glycols, chitosan, or polydimethylamine, or a combination thereof.

It is to be understood that each of the embodiments described above for the first aspect can be combined to define the specific characteristics of the population of MNPs according to the first aspect. For instance, in an embodiment, the population of magnetic nanoparticles (MNPs) are characterized by having an iron oxide core and:

i) having an average particle diameter of between 8-15 nm, being coated APTES and having a magnetic anisotropy of preferably 27 mT ± 25% (e.g. CSIC10);

ii) having an average particle diameter of between 8-15 nm, being coated aminated-dextran (DEX) and having a magnetic anisotropy of preferably 27 mT ± 25% (e.g. CSIC13).

Step b) of the method may be performed previously, simultaneously, or subsequently to step a).

Step c) of the method of the present invention comprises immobilizing the MNPs in the porous scaffold. This is performed by contacting the MNPs with the porous scaffold under suitable conditions for the immobilization of the MNPs to the scaffold. Preferably, the immobilization of the MNPs to the scaffold is via covalent bonds.

MNP covalent immobilization within the porous scaffold is carried out at conditions to ensure either their heterogenous or homogenous distribution of the MNPs thorough the porous scaffold.

For achieving an heterogenous integration, the immobilization of the aminated MNPs on the aldehyde scaffold, preferably on the aldehyde agarose microbeads, is carried out at room temperature (between 25-20 °C) at an alkaline pH (preferably ranging between 8-10, being 10 usually preferred binding pH) using suitable buffers (preferably Na-bicarbonate, Na-borate, CAPS), a high density of aldehyde groups at the scaffold. The preferred range of aldehyde groups at the scaffold is between 15-100, 20-80, 25-70, preferably 25-60 $\mu$mol aldehyde/mg of wet scaffold. The preferred range of amino groups MNPs with a range of amino groups from 30 to 100, preferably 40 to 84 $NH_2/mg_{Fe}$.

In a preferred embodiment, the porous scaffold comprises agarose microbeads and is coated and/or activated to provide on its surface carboxyl functional groups, wherein the MNPs are coated and/or activated to provide on their surface amino functional groups, and wherein the immobilization of step c) is a heterogenous immobilization carried out at a temperature of between 25-20°C, pH ranging between 8-10, and during periods of time between 30 minutes to 1h, and using a suitable buffer, wherein the reaction is carried according to the following steps:

a) adding the aldehyde agarose beads to the aminated MNPs dispersions and incubate for at least 30 minutes up to 1 h at a temperature of between 25-20°C,
b) reducing the Schiff's bases to secondary amino groups and optionally the remaining aldehyde groups into hydroxyl groups by adding a suitable reducing agent such as sodium borohydride,
c) optionally washing the MNPs immobilized to the agarose beads,

wherein preferably, after MNPs immobilization, derivatives are reduced with a suitable reducing agent such as sodium borohydride in order to reduce Schiff's bases to secondary amino groups and to reduce remaining aldehyde groups into hydroxyl ones, and wherein after MNPs immobilization, the MNPs are preferably activated to functionalize their surface with at least one biomolecule, by covalent bonds or metal affinity.

Preferably, the MNPs are coated and/or activated to provide amino functional groups, the porous support comprises agarose microbeads and is coated and/or activated to provide carboxyl functional groups, and wherein the immobilization of step c) is a heterogenous immobilization carried out at a temperature of between 25-20°C, pH ranging between 8-10, and during periods of time greater than 30 minutes and up to 1 h, and using a suitable buffer, wherein the reaction is carried according to the following steps:

a) washing the aldehyde agarose beads with the buffer in which the aminated MNPs will be suspended for their immobilization to the microbeads,
b) sonicating the MNPs in a glass flask with the immobilization buffer,
c) adding the aldehyde agarose beads to the aminated MNPs dispersions and incubate the mix by end-over-end rotation for at least 30 minutes up to 1 h at room temperature,
d) reducing the Schiff's bases to secondary amino groups and the remaining aldehyde groups into hydroxyl ones by adding a suitable reducing agent such as sodium borohydride,
e) washing the hybrids with sodium bicarbonate buffer 25 mM at pH 8.5 and store them filtered dried under vacuum until use for enzyme binding,

wherein after MNPs immobilization, derivatives are reduced with a suitable reducing agent such as sodium borohydride in order to reduce Schiff's bases to secondary amino groups and to reduce remaining aldehyde groups into hydroxyl ones, and wherein after MNPs immobilization, derivatives are activated to conduct the binding of a biomolecule of interest, preferable at least one enzyme, by covalent bonds or metal affinity.

In a preferred embodiment, the heterogenous MNPs covalent integration is carried out at 25 °C in 0.01 M Na-borate buffer pH 8.0 or 0.1 M Na-bicarbonate buffer pH 10.0 and 0.1% CTAB;

For achieving a homogenous integration, the immobilization is carried out at temperature range of less than 10°C, preferably at about 4 °C, at an alkaline pH, preferably at a pH ranging between 7-9 (being 8.5 usually preferred binding pH), and using:

i) suitable buffers (Na-bicarbonate, Na-borate, HEPES),
ii) a broad range of aldehyde groups at the agarose microbeads (from 4 to 52),

iii) Schiff's base stabilizing agents (eg. Sodium borohydride, DTT, mercaptoethanol),
iv) aminated molecules as competitors for agarose aldheyde's groups to slow down the kinetics of immobilization (eg. ethylenediamine, ethanolamine, glycine, etc); and
v) surfactants to improve colloidal stability of aminated MNPs over long incubation times (>24 h) (eg. CTAB); and vi) MNPs with a range of amino groups from 15 to 35 $NH_2/mg_{Fe}$.

In a preferred embodiment, the porous scaffold comprises agarose microbeads and is coated and/or activated to provide on its surface carboxyl functional groups, the MNPs are coated and/or activated to provide on their surface amino functional groups, and the immobilization of step c) is a homogeneous immobilization carried out at a temperature of less than 10°C, at a pH ranging between 7-9 and during periods of between 24h and 72h, and using:

i) a buffer,
ii) aldehyde agarose beads having from 4 to 52 $\mu$mol of aldehydes groups/wet gram of agarose,
iii) Schiff's base stabilizing agents,
iv) aminated molecules as competitors for agarose aldehyde's groups to slow down the kinetics of immobilization; and
v) surfactants; and
vi) MNPs with a range of amino groups from 159 to 352 $NH_2/mg$ Fe,

wherein the reaction is carried according to the following steps:

d) adding the agarose beads to the aminated MNPs dispersions and incubate the mix for at least 24 h up to 72h at an incubation temperature lower than 10°C,
e) reducing the Schiff's bases to secondary amino groups and the remaining aldehyde groups into hydroxyl ones by adding a suitable reducing agent such as sodium borohydride in order to reduce Schiff's bases to secondary amino groups and to reduce remaining aldehyde groups into hydroxyl ones.
f) optionally, washing the MNPs immobilized to the agarose beads,

wherein preferably, after MNPs immobilization, derivatives are reduced with a suitable reducing agent such as sodium borohydride in order to reduce Schiff's bases to secondary amino groups and to optionally reduce remaining aldehyde groups into hydroxyl ones,
wherein after MNPs immobilization, the MNPs are preferably activated to functionalize their surface with at least one biomolecule, by covalent bonds or metal affinity.

In a preferred embodiment, the porous scaffold comprises agarose microbeads and is coated and/or activated to provide on its surface carboxyl functional groups, the MNPs are coated and/or activated to provide on their surface amino functional groups, and the immobilization of step c) is a homogeneous immobilization carried out at a temperature of less than 10°C, at a pH ranging between 7-9 and during periods of between 24h and 72h, and using:

i) a buffer,
ii) aldehyde agarose beads having from 4 to 52 $\mu$mol of aldehydes groups/wet gram of agarose,
iii) Schiff's base stabilizing agents,
iv) aminated molecules as competitors for agarose aldehyde's groups to slow down the kinetics of immobilization; and
v) surfactants; and
vi) MNPs with a range of amino groups from 159 to 352 $NH_2/mg$ Fe,

wherein the reaction is carried according to the following steps:

a) washing the aldehyde agarose microbeads with the buffer in which the aminated MNPs will be suspended for their immobilization to the microbeads,
b) sonicating the MNPs, preferably in a glass flask, with an immobilization buffer,
c) adding the aldehyde agarose beads to the aminated MNPs dispersions and incubate the mix by end-over-end rotation for at least 24 h up to 72h at an incubation temperature lower than 10°C, preferably 4°C,
d) reducing the Schiff's bases to secondary amino groups and the remaining aldehyde groups into hydroxyl ones by adding a suitable reducing agent such as sodium borohydride,
e) washing the hybrids with sodium bicarbonate buffer, preferably at a concentration of 25 mM at pH 8.5, and store them filtered dried under vacuum until biomolecule functionalization,

wherein after MNPs immobilization, derivatives are reduced with a suitable reducing agent such as sodium borohydride in order to reduce Schiff's bases to secondary amino groups and to reduce remaining aldehyde groups into hydroxyl ones, wherein after MNPs immobilization, the MNPs are preferably activated to functionalize their surface with at least one biomolecule, by covalent bonds or metal affinity.

Preferably, the reduction step in both homogeneous and heterogeneous immobilization is carried out adding to the MNPs immobilized to the agarose microbeads 1mg/mL of NaBH4 for 30 minutes at room temperature.

In a preferred embodiment, the homogenous MNPs covalent integration is carried out at at 4 °C in 0.1 M Na-bicarbonate buffer pH 8.5 or 0.1M HEPES pH 7.5, 0.1% CTAB and 50 mM ethylenediamine.

The porous scaffold is thus used to integrate MNPs, to tune their magnetic heating efficiency. In an embodiment, the MNPs are coated (including functionalized with at least one biomolecule) before their immobilization in the porous scaffold. In an alternative embodiment, the MNPs are coated (including functionalized with at least one biomolecule) after their immobilization in the porous scaffold. It is preferably that the MNPs are functionalized with at least one biomolecule after their immobilization in the porous scaffold, i.e., after step c). Thus, in a preferred embodiment, the process comprises a further step d) comprising contacting the MNPs with a biomolecule under suitable conditions for the immobilization of the biomolecule to the MNPs by means of covalent bonds or meta affinity interactions.

In an embodiment, the MNPs comprise or are coated and/or activated to provide hydroxyl, carboxyl, carbonyl, amino, thiol, azide, or alkyne functional groups before their immobilization in the porous scaffold, and the MNPs are functionalized with at least one biomolecule, preferably an enzyme, after immobilization to the porous scaffold. In an embodiment, previously or subsequently to step c), a biomolecule, preferably an enzyme, is immobilized on the surface of the porous scaffold.

In an embodiment, the process of the first aspect comprises the steps of:

a) providing a porous scaffold, wherein the porous scaffold comprises or is activated to provide on its surface hydroxyl, carboxyl, carbonyl, amino, thiol, azide, or alkyne functional groups capable of covalently binding MNPs and optionally biomolecules to said porous scaffold;

b) providing at least a population of magnetic nanoparticles (MNPs), characterized in that the MNPs have a magnetic anisotropy capable of dissipating local thermal energy on their surface upon application of an external alternating magnetic field (AMF), wherein the MNPs comprise or are activated to provide on their surface hydroxyl, carboxyl, carbonyl, amino, thiol, azide, or alkyne functional groups capable of covalently binding the porous scaffold of step a), and to the biomolecules,

c) contacting the MNPs of b) with the porous microbeads of a) under suitable conditions for the immobilization of the MNPs to the microbeads by means of covalent bonds, and

d) contacting the immobilized MNPs with a biomolecule under suitable conditions for the immobilization of the biomolecule to the MNPs by means of covalent bonds or affinity metal interactions.

In an embodiment, the process of the first aspect comprises the steps of:

a) providing a scaffold comprising porous microbeads, wherein the microbeads comprise or are coated and/or activated to provide hydroxyl, carboxyl, carbonyl, amino, thiol, azide, or alkyne functional groups capable of covalently binding MNPs and optionally biomolecules to said porous scaffold,

b) providing a population of MNPs, wherein the MNPs comprise or are coated and/or activated to provide hydroxyl, carboxyl, carbonyl, amino, thiol, azide, or alkyne functional groups capable of covalently binding the porous scaffold of step a) and to the biomolecules,

c) contacting the porous microbeads of a) with the MNPs of b) to immobilize the MNPs by covalent bonds to the coated porous microbeads,

d) activating the integrated MNPs to ensure the site-directed of biomolecule, preferably an enzyme, to the surface of the MNPs by means of covalent bonds or metal affinity, and contacting the MNPs with a biomolecule, preferably an enzyme, under suitable conditions for the immobilization of the biomolecule to the MNPs by means of covalent bonds or metal affinity interactions,

wherein steps a) and b) can be performed in any order.

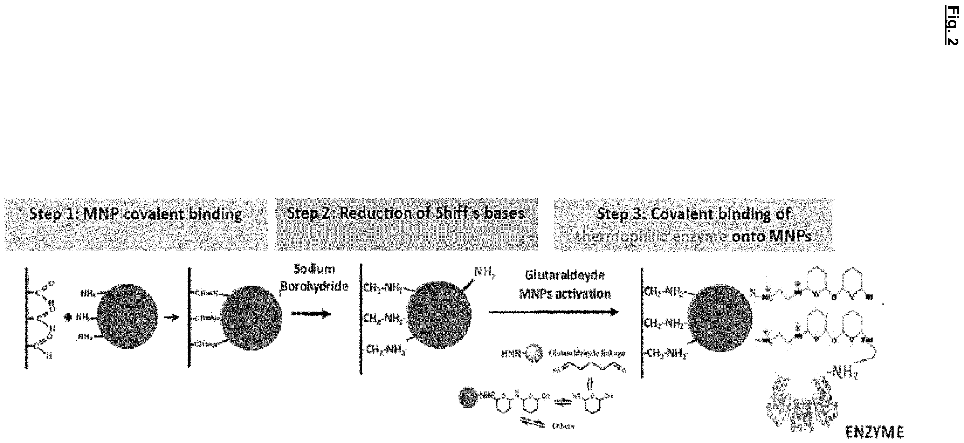

In an embodiment, the process of the first aspect comprises the steps of (see Fig. 2):

a) providing a scaffold comprising porous microbeads, wherein the microbeads comprise or are coated and/or activated to provide carbonyl functional groups, preferably aldehyde functional groups, capable of covalently binding MNPs and optionally biomolecules to said porous scaffold,

b) providing a population of MNPs, wherein the MNPs comprise or are coated and/or activated to provide amino functional groups capable of covalently binding the porous scaffold of step a) and to the biomolecules,

c) contacting the carbonyl porous microbeads with the amino MNPs to immobilize the amino MNPs by covalent bonds to the carbonyl porous microbeads,

d) activating the immobilized MNP with amine-reactive crosslinkers, preferably glutaraldehyde, and contacting the MNPs with a biomolecule, preferably an enzyme, under suitable conditions for the immobilization of the biomolecule to the MNPs by means of covalent bonds or metal affinity interactions,

wherein steps a) and b) can be performed in any order, and wherein step c) is preferably carried out by homologous or heterologous immobilization methods, as defined above.

In an embodiment, the process of the first aspect comprises the steps of:

a) providing a scaffold comprising porous microbeads, wherein the microbeads comprise or are coated and/or activated to provide hydroxyl, carboxyl, carbonyl, amino, thiol, azide, or alkyne functional groups capable of covalently binding MNPs and optionally biomolecules to said porous scaffold,
b) providing a population of MNPs, wherein the MNPs comprise or are coated and/or activated to provide hydroxyl, carboxyl, carbonyl, amino, thiol, azide, or alkyne functional groups capable of covalently binding the porous scaffold of step a) and to the biomolecules,
c) contacting the porous microbeads of a) with the MNPs of b) to immobilize the MNPs by covalent bonds to the porous microbeads,
d) contacting the MNPs with a biomolecule, preferably an enzyme, under suitable conditions for the immobilization of the biomolecule to the MNPs by means of covalent bonds or metal affinity interactions, and contacting the scaffold with a biomolecule, preferably an enzyme, under suitable conditions for the immobilization of the biomolecule, preferably an enzyme, to the porous microbeads by means of covalent bonds or metal affinity interactions

wherein steps a) and b) can be performed in any order, and wherein step c) is preferably carried out by homologous or heterologous immobilization methods, as defined above.

In an embodiment, the process of the first aspect comprises the steps of (see also Fig 30):

a) providing a scaffold comprising porous microbeads, wherein the porous microbeads comprise or are coated and/or activated to provide carbonyl functional groups, preferably aldehyde functional groups, capable of covalently binding MNPs and optionally biomolecules to said porous scaffold,
b) providing a population of MNPs, wherein the MNPs comprise or are coated and/or activated to provide amino functional groups capable of covalently binding the porous scaffold of step a) and to the biomolecules,
c) contacting the carbonyl porous microbeads of a) with the amino MNPs of b) to immobilize the MNPs by covalent bonds to the coated porous microbeads,
d) activating the immobilized MNP with amine-reactive crosslinkers, preferably glutaraldehyde, and contacting the MNPs with a biomolecule, preferably an enzyme, under suitable conditions for the immobilization of the biomolecule to the MNPs by means of covalent bonds or metal affinity interactions,
e) functionalizing the carbonyl porous microbeads with at least one biomolecule, preferably at least one enzyme, and
f) optionally, reducing Shiff's bases,

wherein steps a) and b) can be performed in any order, and wherein step c) is preferably carried out by homologous or heterologous immobilization methods, as defined above.

In an embodiment, the system is obtained by contacting MNPs with a porous scaffold, wherein both the porous scaffold and the MNPs comprise activated functional groups, preferably selected from the list consisting of hydroxyl, carboxyl, carbonyl, amino, thiol, azide, or alkyne functional groups, wherein the contact of the MNPs and the porous scaffold results in the immobilization of the MNPs to the porous scaffold by means of covalent bonds (preferably carboxyl-amino, as explained above). Next, the immobilized MNPs are activated and functionalized with at least one biomolecule, preferably an enzyme, wherein:

- if the functionalization is carried out by covalent bounds (i.e., "direct functionalization") the activation of the MNPs is performed with glutaraldehyde, or
- if the functionalization is carried out by metal affinity interactions (i.e., indirect functionalization), the activation of the MNPs is performed with glutaraldehyde and NTA-Me$^{+2}$, and wherein the enzyme comprises at least one His-Tag.

In **a second aspect,** the present invention provides a system of MNPs immobilized on a porous scaffold, obtained or obtainable from the process of the first aspect.

In **a third aspect,** the present invention provides a system of MNPs immobilized on a porous scaffold, wherein the MNPs and the scaffold are as defined in the first aspect or any of its embodiments and thus said embodiments also apply herein.

Preferably, the system comprises:

a) a porous scaffold, wherein the porous scaffold is characterized by comprising or being coated and/or activated to provide on its surface at least one functional group capable of covalently binding MNPs to said porous scaffold, wherein said at least one functional groups are selected from the list consisting of hydroxyl, carboxyl, carbonyl, amino, thiol, azide, or alkyne functional groups.
b) a population comprising MNPs, characterized in that the MNPs have a magnetic anisotropy capable of dissipating local thermal energy on their surface upon application of an external alternating magnetic field (AMF), wherein the MNPs are further characterized comprising or being coated and/or activated to provide on its surface at least one functional group capable of covalently binding said MNPs to the porous scaffold of a), wherein said at least one functional group is selected from the list consisting of hydroxyl, carboxyl, carbonyl, amino, thiol, azide, or alkyne groups.

Preferably, the population comprising MNPs is characterized in that the MNPs have a magnetic anisotropy capable of dissipating local thermal energy on their surface upon application of an external alternating magnetic field (AMF), wherein the MNPs are further characterized by having an iron oxide core and being selected from the list consisting of:

> i) having an average particle diameter of between 8-15 nm, being coated APTES and having a magnetic anisotropy of 34 ± 50%, preferably 27 mT ± 25%,
>
> ii) having an average particle diameter of between 8-15 nm, being coated aminated-dextran (DEX) and having a magnetic anisotropy of 34 ± 50%, preferably 27 mT ± 25% .

In a **fourth aspect,** the present invention provides the use, preferably the *in vitro* use, of the system of the second or third aspect, or any of their embodiments, for carrying out at least an enzymatic reaction. Said enzymatic reaction may be useful in biosensing applications, or in biotechnology-based applications such as enzyme based therapies (e.g. enzyme replacement treatment, enzyme prodrug or direct therapy, enzyme dynamic or starving therapy) or the implementation of complex biotransformations based on the use of enzymatic cascades for the production of high-cost pharmaceuticals, health supplements, agrochemicals, biofuel to low-cost biocommodities (eg. hydrogen, alcohols).

In **a fifth aspect,** the present invention provides a method for carrying out an enzymatic reaction, the method comprising the steps of:

> a) providing the system of the second or third aspect, or performing the process of the first aspect to provide the system of the invention,
>
> b) applying one or more external AMFs to the system of a) to produce the activation of the at least one enzyme functionalized on the MNPs so that the enzymatic reaction is carried out.

By "external alternating magnetic field" (AMF) is meant Alternating magnetic fields (AMFs) that causes magnetic nanoparticles (MNPs) to dissipate heat. In an embodiment, the local thermal energy dissipated on the surface of each MNP upon application of an external AMF is sufficient to activate and/or inactivate the at least one enzyme functionalized on the surface of said MNP. In a preferred embodiment, the dissipated thermal energy on the surface of the MNPs is within the range of from 30-90 °C.

In an embodiment, the external AMF comprises a parameter of magnetic flux density and a parameter of frequency. The magnetic flux density or magnetic induction, measured in tesla (T), is the number of field lines passing through a unit area of material. It is noted that terms "magnetic field", "magnetic flux" and "flux density" are often used as synonyms.

The characteristics of the population of MNPs as defined above in the first aspect or in any of its embodiments can be combined with any of the external AMF settings (magnetic flux density and frequency values) that are detailed below.

In an embodiment, the external AMF is characterized by a magnetic flux density applied in the ranges from 1 to 100 mT. In another embodiment, the external AMF is characterized by a magnetic flux density applied in the ranges from 5 to 90, 5 to 80, 5 to 70, 5 to 60, 5 to 50, 5 to 40, 5 to 35, 5 to 30, 5 to 25, 5 to 20, 5 to 15, 5-10 or 1-10 mT. In a further embodiment, the external AMF is characterized by a magnetic flux density applied in the ranges from 5 and 70 mT. In a preferred embodiment, the external AMF is characterized by a magnetic flux density applied in the ranges from 5-20, 10-20, 20-30, 30-40, 40-50, 50-60 or 60-70 mT.

By "frequency" is meant the number of cycles per second (from maximum positive to maximum negative and back) of the magnetic field. The more cycles that occur per second, the higher the frequency. In an embodiment, the external AMF is characterized by a frequency applied in the ranges from 1 to 1000 kHz. In another embodiment, the external AMF is characterized by a frequency applied in the ranges from 1 to 900, 1 to 800, 1 to 700, 1 to 600, 1 to 500, 1 to 400, 1 to 300, 1 to 200, 1 to 100, 1 to 750, or 1 to 50 kHz. In a preferred embodiment, the external AMF is characterized by a frequency applied in the ranges from 50 to 800 kHz. In a further preferred embodiment, the external AMF is characterized by a frequency applied in the ranges from 50 and 800 kHz. In an embodiment, an external AMF of low frequency refers to a frequency applied of less of 100 kHz. In an embodiment, an external AMF of medium frequency refers to a frequency applied that ranges from 100 to 400 kHz. In an embodiment, an external AMF of high frequency refers to a frequency applied that is higher than 400 kHz.

It is noted that, in order to define the external AMF applied, any combination of the magnetic flux density ranges or values with the frequency range or values as defined above can be combined. Thus, in an embodiment, the external AMF applied can be selected from the group consisting of i) magnetic flux density range of 40-50 mT and frequency range of 80-120 kHz; ii) magnetic flux density range of 5-20 mT, and frequency range of 300-400 kHz; iii) magnetic flux density range of 10-20 mT, and frequency range of 400-500 kHz; iv) magnetic flux density range of 50-60 mT, and frequency range of 50-100 kHz; v) magnetic flux density range of 50-60 mT, and frequency range of 50-100 kHz; vi) or magnetic flux density range of 20-30 mT, and frequency range of 600-800 kHz; vii) magnetic flux density range of 5-20 mT, and frequency range of 600-800 kHz.

Therefore, in an embodiment, one or more external AMFs are applied to the populations of MNPs. In a preferred embodiment, the process of the fifth aspect of the invention is to carry out two or more simultaneous enzymatic reactions, wherein the external AMFs applied in step b) of the fifth aspect is sufficient to produce the simultaneous activation of different enzymes functionalized on the surface of the populations of MNPs and, optionally, on the surface of the scaffold.

In a preferred embodiment, the process of the fifth aspect of the invention is to carry out two or more sequential enzymatic reactions, wherein the one or more applied external AMFs of step b) of the fifth aspect are applied sequentially so as to produce the sequential activation of different enzymes functionalized on the surface of the populations of MNPs and, optionally, on the surface of the scaffold.

It is important to define the conditions of applied field (Hmax) and magnetic field anisotropy (HA) that maximize the heat dissipation. The best strategy to maximize the SAR is to maximize the anisotropy of the system up to the point in which the applied field is able to perform major loops.

In an embodiment, the value of the external field in relation with HA is considered. In another embodiment, the ratio between HMAX and HA is taken as criteria to predict the heating power of MNPs at a certain applied field. In a further embodiment, for a randomly distributed non-interacting system the hysteresis losses will be negligible for Hmax/HA < 0.5 [Munoz-Menendez et al. 2017].

Thus, in the context of the present invention, the following can be considered:

- Negligible dipolar interactions between magnetic nanoparticles (assured by the colloidal stability of the suspensions, only achieved after proper coating).
- All the heat dissipation mechanisms are included in the magnetic loops (no heating from the hyperthermia equipment).
- Anisotropy of the nanoparticles are defined by the contribution of shape and crystal (surface anisotropy is considered negligible for particle sizes over 10 nm where surface represents less than 25% of the volume).

In an embodiment, the value ratio Hmax/HA comprises any value in between 0.4 and 1.5. In an embodiment, the ratio between the magnetic flux density and the magnetic anisotropy is greater than 0.4. In a preferred embodiment, the ratio between the magnetic flux density and the magnetic anisotropy is greater than 0.5. In another embodiment, the ratio between the magnetic flux density and the magnetic anisotropy is between 0.4 and 1.2. In a preferred embodiment, the ratio between the magnetic flux density and the magnetic anisotropy is between 0.5 and 1.1.

In another embodiment, the magnetic anisotropy of the populations of MNPs is within the range of 34 mT $\pm$ 50%, and the external AMF is characterized by a frequency range between 50 and 800 kHz and a magnetic flux density of between 5 and 70 mT, wherein, preferably, the ratio between the magnetic flux density and the magnetic anisotropy is greater than 0.4, preferably greater than 0.5. In a preferred embodiment, the magnetic anisotropy of the populations of MNPs is within the range of 34 mT $\pm$ 50%, and the external AMF is characterized by a frequency range between 50 and 800 kHz and a magnetic flux density between 5 and 70 mT, and the ratio between the magnetic flux density and the magnetic anisotropy is between 0.4 and 1.2. In an even more preferred embodiment, the magnetic anisotropy of the populations of MNPs is within the range of 34 mT $\pm$ 50%, and the external AMF is characterized by a frequency range between 50 and 800 kHz and a magnetic flux density of between 5 and 70 mT, and the ratio between the magnetic flux density and the magnetic anisotropy is between 0.5 and 1.1.

In a preferred embodiment, the population of MNP comprised in the system of the fourth aspect are characterized by having a particle-size distribution of less than 0.5, preferably less than 0.1, an iron oxide core, and:

i) an average particle diameter of between 8-15 nm, are coated with DMSA, functionalized with NTA and a divalent ion, preferably $Cu^{2+}$, and have a magnetic anisotropy of 34 $\pm$ 25%, preferably 27 mT;
ii) an average particle diameter of between 18-25 nm, are coated with PMAO, functionalized with NTA and a divalent ion, preferably $Cu^{2+}$, and have a magnetic anisotropy of 34 $\pm$ 25%, preferably 34 mT; or
iii) an average particle diameter of between 25-35 nm, are coated with PAA, functionalized with NTA and a divalent ion, preferably $Cu^{2+}$, and have a magnetic anisotropy of 34 $\pm$ 25%, preferably 41 mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz, v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

In a preferred embodiment, the MNPs are characterized by:

- having an average particle diameter of between 8-15 nm, being coated APTES and having a magnetic anisotropy of 34 $\pm$ 50%, preferably 27 mT $\pm$ 25% (eg CSIC10, see Examples);

- having an average particle diameter of between 8-15 nm, being coated aminated-dextran (DEX) and having a magnetic anisotropy of 34 ± 50%, preferably 27 mT ± 25% (CSIC13, see Examples),

and wherein the one or more AMF applied are selected from the group consisting of:

i) magnetic flux density range of 30 mT and frequency range of 150-700 kHz (e.g. CSIC10 MNPs) or
ii) a magnetic flux density range of 16 mT and a frequency of 300 kHs (e.g. CSIC13 MNPs).

In a preferred embodiment of the fifth aspect, the method comprises the steps of:

a) providing a porous scaffold as defined in the first aspect or any of its embodiments,

b) providing at least a population of magnetic nanoparticles (MNPs), as defined in the first aspect or any of its embodiments,

c) contacting the MNPs with the porous scaffold under suitable conditions for the immobilization of the MNPs to the scaffold by means of covalent bonds, as defined in the first aspect or any of its embodiments,

d) contacting the MNPs with a biomolecule under suitable conditions for the immobilization of the biomolecule to the MNPs by means of covalent bonds or affinity metal interactions, as defined in the first aspect or any of its embodiments,

e) applying one or more external AMFs to the system resulting from step d) to produce the activation of the enzyme functionalized on the MNPs so that the enzymatic reaction is carried out, as defined in the fifth aspect or any of its embodiments,

wherein steps a) and b) can be performed in any order, and wherein step c) is preferably carried out by homologous or heterologous immobilization methods, as defined above.

In a preferred embodiment of the fifth aspect, the method comprises the steps of:

a) providing a porous scaffold, wherein the porous scaffold comprises or is coated and/or activated to provide on its surface hydroxyl, carboxyl, carbonyl, amino, thiol, azide, or alkyne functional groups capable of covalently binding MNPs to said porous scaffold;

b) providing at least a population of magnetic nanoparticles (MNPs), characterized in that the MNPs have a magnetic anisotropy capable of dissipating local thermal energy on their surface upon application of an external alternating magnetic field (AMF), wherein the MNPs comprise or are coated and/or activated to provide on their surface hydroxyl, carboxyl, carbonyl, amino, thiol, azide, or alkyne functional groups capable of covalently binding the porous scaffold of step a),

c) contacting the MNPs of b) with the porous scaffold of a) under suitable conditions for the immobilization of the MNPs to the scaffold by means of covalent bonds,

d) contacting the MNPs with a biomolecule under suitable conditions for the immobilization of the biomolecule to the MNPs by means of covalent bonds or affinity metal interactions,

e) applying one or more external AMFs to the system resulting from step d) to produce the activation of the enzyme functionalized on the MNPs so that the enzymatic reaction is carried out,

wherein steps a) and b) can be performed in any order, and wherein step c) is preferably carried out by homologous or heterologous immobilization methods, as defined above.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

The invention is described below by the following examples, which must be considered as merely illustrative and in no case limiting of the scope of the present invention.

**EXAMPLES**

**1) MICROHYBRIDS: TUNING MAGNETIC HEATING OF MNPs WITHOUT TUNING MNPs ANISOTROPY**

1.1)- TUNING MAGNETIC HEATING BY CONTROLLING THE SPATIAL DISTRIBUTION OF MNPs.

First, we focused on the integration of the MNPs to achieved different hierarchical distributions of the MNPs within agarose microbeads structure. It has been proved that it is possible to tune the rate of immobilization of the MNPs to obtain a homogenous or heterogenous distribution of the MNPs into the porous beads. Our hypothesis is that the difference in the MNPs distribution could affect the efficiency of the thermal activation of the enzyme attached to surface of the MNPs (previously integrated within the microbead structure) triggered by AMF application (Figure 1). This way, spatial location of the MNPs could be an additional mechanism for tuning the local temperature gradient achieved at the enzyme microenvironment. In fact, the heterogeneous distribution of MNPs, correspond to a high concentration of the particles within a concrete region near the surface of the microbeads, and we hypothesize that this distribution could generate high temperature gradients at the microscale that at certain AMF application conditions could even affect the global temperature of the reaction media. On the other hand, homogeneous distribution, where the MNPs are evenly separated within the microbead structure and thus magnetically isolated from each other, would allow triggering a very localized temperature gradient at the nanoscale without having influence in the bulk temperature.

The preparation of Microhybrids (mHs) has been carried by optimizing the direct covalent conjugation of MNPs (12 nm) containing amino groups to microporous agarose beads (50-200 $\mu$m) containing aldehyde groups. The immobilization of aminated-MNPs on glyoxyl-agarose occurs spontaneously via a multipoint-point simultaneous attachment via Schiff's base formation (imine bond). These reversible Schiff's bases are stabilized into irreversible secondary amine bonds via reduction using sodium borohydride as reducing agent. Besides, all the remaining aldehyde groups not used on the immobilization process are also reduced to alcohol moieties ensuring the scaffold inertness. Then, the site-directed binding of the enzyme to be nanoactuated onto the surface of the MNPs is carried out by activating the MNP's amine moieties with glutaraldehyde (Figure 2).

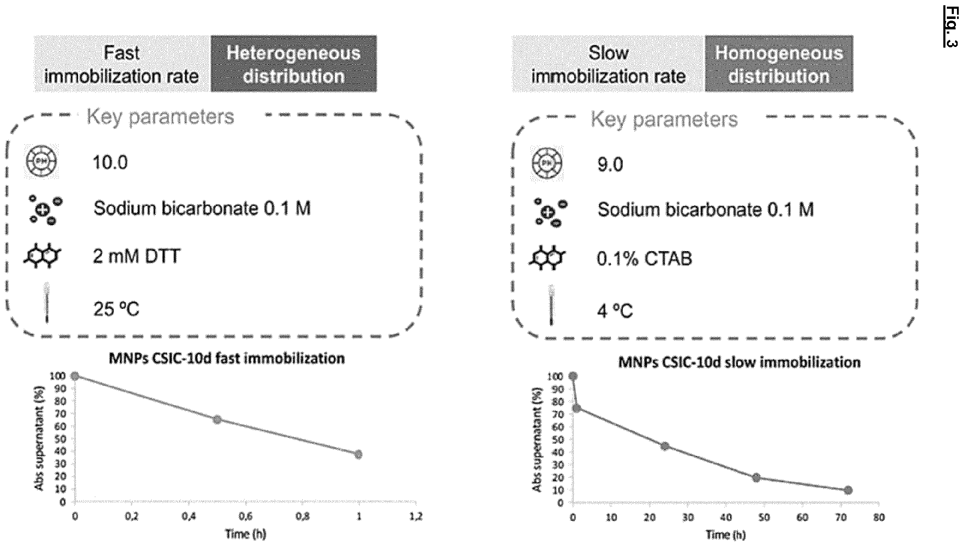

To achieve a differential spatial distribution of MNPs during their covalent binding, the control of the kinetic of their covalent immobilization has been identified as key point. So, to achieve a fast or slow immobilization, key parameters have been defined: ionic strength, pH, amount of CTAB, amount of ethylenediamine, temperature, density of amine groups at the MNP surface and density of aldehyde groups at the agarose beads (Figure 3).

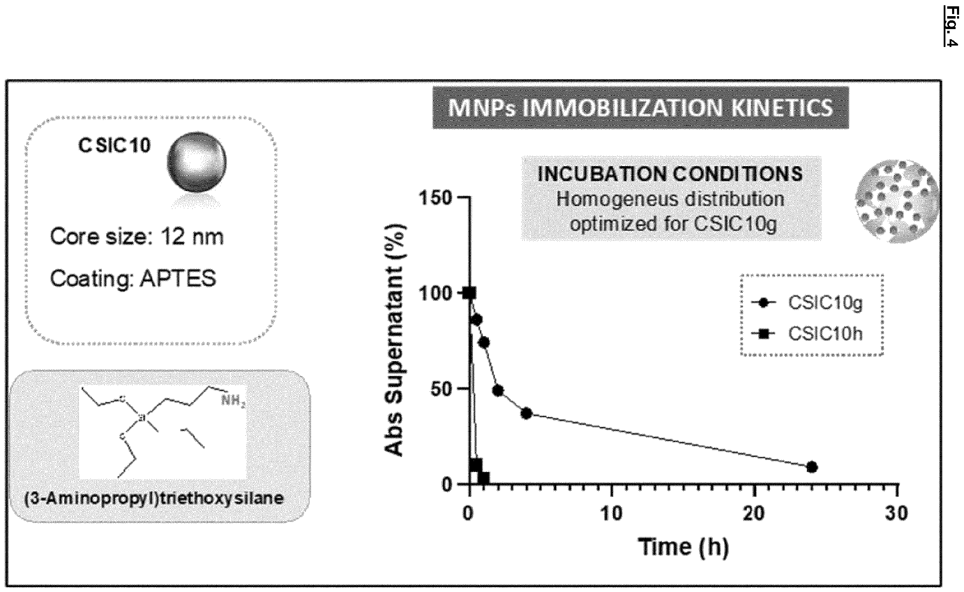

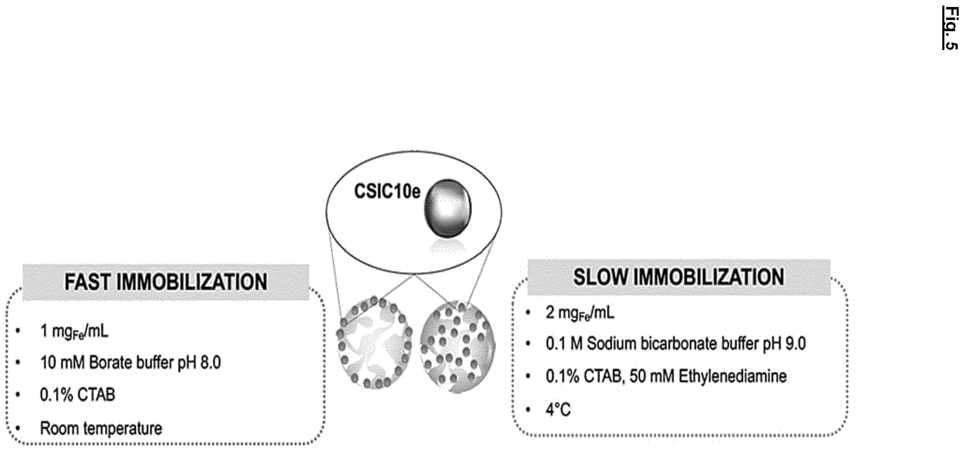

Adding thiolated compounds as additives in the immobilization media allowed to speed up the immobilization kinetics as the unstable Schiff's bases formed between one amino group and one glyoxil group are stabilized by thiolated compounds. Thus, in the presence of thiolated compounds such as dithiothreitol (DTT), a stable binding of the MNPs with the carrier may also occur via a one-point covalent attachment. If, on the contrary, what is wanted is to promote a slow immobilization of the MNPs, several parameters could be tuned such as decreasing the concentration of available aldehyde groups on the agarose or amine-groups at the MNP surface. The density of amine groups at the MNP surface is another critical parameter to tune MNPs binding kinetics (Figure 4). However, even if MNPs are used with different amine-content, it has been shown that it is possible to re-optimize the immobilization conditions to gain again control on their immobilization kinetics by slightly tuning some parameters previously showed that affect MNPs immobilization rate (e.g., CTAB, EDA, pH, ionic strength of the immobilization buffer and temperature) (Figure 5).

Once key parameters to tune MNPs immobilization kinetics was defined, it has been shown that a fast MNP immobilization corresponds to a more heterogeneous distribution, where more than 90% of the nanoparticles are immobilized in less than an hour of incubation within the microbead surface. On the other hand, when the MNP immobilization happens more slowly (around 50% after 2h of incubation and 100% after 24 hours), the particles spatial distribution within the microbeads structure tends to be more homogeneous, being found not only at the outer surface of the microbeads but also in their internal porous structure.

The spatial distribution of the MNPs was observed by confocal microscopy after attaching green fluorescent protein (GFP) to the surface of MNPs integrated either heterogeneously or homogenously (Figure 6). Besides, their direct observation when heterogeneously distributed was optimized, by the inclusion of the obtained hybrids in a methyl-cellulose matrix to obtain thin slices by cutting the samples using a microtome.

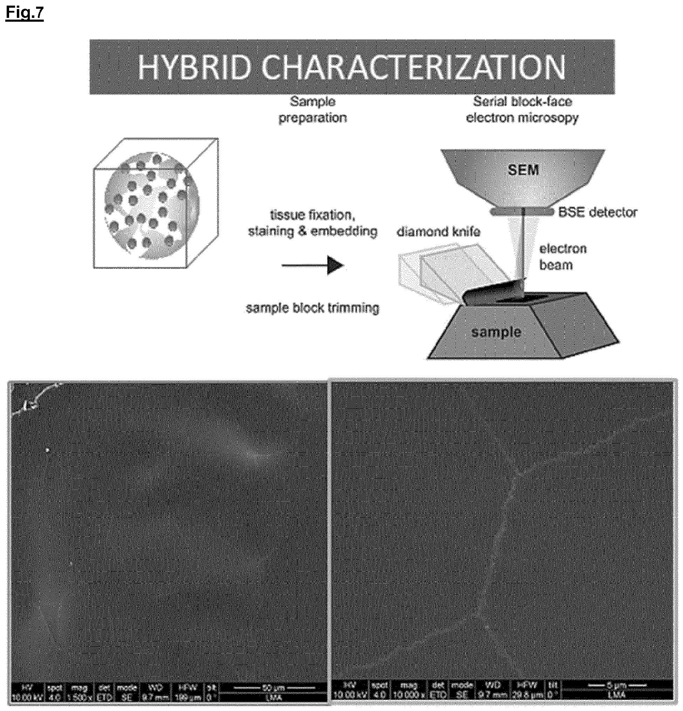

Then the distribution of the MNPs was checked by direct observation using scanning electron microscopy (SEM) (Figure 7).

After, optimizing MNPs immobilization conditions to tune their spatial location within porous beads, the next step was to study how these enzyme/MNP/microbead integration influence the heat transference process both; i) at a macroscopic level by measuring the global media magnetic heating capacity of concentrated mHs samples, and also ii) at the micro and nanoscopic level by assessing the increase on the initial biotransformation rate of the selected enzymes using activity assays with a colorimetric red out.

1.1.1)- Tuning the global heating capacity of mHs by tuning the spatial distribution of the MNPs integrated within the porous beads.

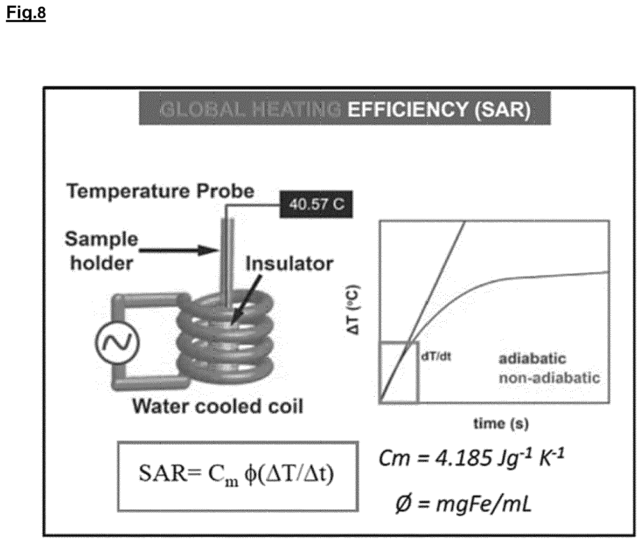

The heat power released to the media were the MNPs are suspended (macroscopic scale) upon application of an alternating magnetic field is quantified by means of the specific absorption rate (SAR). As it could be observed in Figure 8, SAR is defined as the ratio between the heat power dissipated by the MNPs (P) and the mass of magnetic material (MNP).

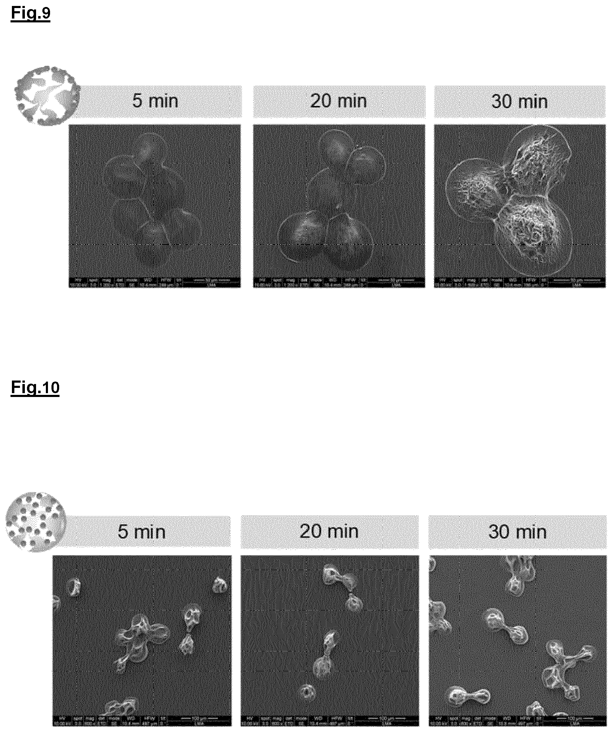

It was observed that rapid immobilizations (around 90% immobilization yield in less than 1 h) located 100% of the loaded MNPs at the crown of the carrier beads, meaning that only around 10% of the agarose bead radius was colonized by the MNPs. Using CSIC10 as model MNPs, such heterogenous distribution led to the generation of a localized hot zone that caused even the collapse of the agarose beads porous structure after short exposure times when AMF of high frequency and filed intensity (772 kHz, 30 mT) was applied (Figure 9). With diluted solutions of these hybrids a raise in the global temperature of the reaction media was observed while AMF was applied (from 19°C to 23°C). However, this increase could not explain the collapse observed of the agarose beads structure as these beads are autoclavable and therefore could resist at least 20 min of incubation at 120 °C. Indeed, no remaining enzyme activity was observed after AMF exposure when these hybrids were functionalized with enzymes (e.g. TLL lipase) that surely is a consequence of both denaturation of the protein structure and collapse of the microbeads structure due the high temperatures reached the microenvironment of the MNPS (microscope temperature) triggered by magnetic heating.

On the contrary, when MNP immobilization was much slower (around 50% after 2h of incubation and 100% between 24-72 hours), a more homogeneous distribution of the MNPs was obtained, resulting in agarose microbeads where MNPs could be localized along the whole diameter of the microbead. Thus, in this case, it could be considered that the MNPs are more thermally isolated from each other. Consequently, as the heat triggered by AMF application is limited to the immediate proximity of the nanoparticle surface, the heating of the microenvironment within the porous structure of the microbeads should need longer AMF application times and/or most extreme magnetic field applied conditions (frequency x field intensity). Indeed, in the case of, the homogenous hybrids obtained, at least no external changes on the microbeads structure were observed (Figure 10). It is important to point out that although the global heating capacity of the MNPs decrease when integrated within the agarose beads compared with the bare MNPs, an increase in the bioconversion rate of those enzymes attached to the MNPs matching the activity displayed at their optimal temperature was able to be triggered by AMF in the case of the homogenous mHs.

These differences in magnetic heating have been also observed on the mHs's capacity of globally heat the water-based media in which they were suspended. The SAR of the obtained hybrids was calculated by a calorimetry method in which heat quantification was performed through the measurement of the global media temperature evolution of a concentrated sample of mHs (1 mg/mL of Fe) under the application of AMF. The SAR value is obtained from initial slope of the curve obtained after plotting global temperature achieved with time. This is usually done with ferrofluids, as mHs tend to settle very fast the specific protocol for their SAR measurement previously optimized was used. *Thermomyces lanuginosus* (TL) lipase microhybrids were prepared as previously optimized. After the immobilization of CSIC10e MNPs on agarose porous microbeads using the protocol to achieve a fast (heterogenous mHs) or slow (homogenous mHs) immobilization, their activation with glutaraldehyde was carried out and finally the covalent binding of TLL or ADH on immobilized particles surface was optimized. Then, SAR values were obtained after applying different AMF combinations of frequency (kHz) and field intensity (mT) (Figure 11). As it could be observed although using mHs dispersions having the same total amount of Fe, heterogenous mHs has a clear higher magnetic heating capacity when high AMF conditions are applied.

A clear reduction of the SAR value of CSIC10 MNPs is observed when the MNPs are covalently integrated within the agarose beads. Although this happened with both types of the obtained hybrids, a differential behaviour is observed between those in which MNPs are spatially located near the outer surface of the agarose bead (heterogenous mHs), with respect to those in which the MNPs are more evenly homogenously spatially distributed within the agarose bead structure (homogenous mHs). Thus, these measurements also confirm a differential location of the MNPs tuned by their immobilization kinetics. These, differential behaviours on their heat transference process at the macroscopic level is also accompanied with differences between them also at the micro/nanoscopic level.

1.2.2)- Tuning the local heating of enzymes attached on the surface of MNPs with different spatial location.

Owing to the way SAR is determined, results relate to MNPs capacity to increase the global temperature of the media in which mHs are suspended (macroscopic heating) but it fails to measure the heat gradient generated in close vicinity of the MNPs (local heating) upon field exposure. Thus, the AMF-tunability of enzymes attached to the MNPs previously integrated within the hybrids was studied by comparing the bioconversion activity of the hybrids with and without AMF application. To determine this, the initial rates for the biotransformation of the corresponding specific substrates was determined after the application of a 5-minute AMF cycle varying the combination of frequency/field intensity used. Thus first, the mHs activity vs temperature profile was assessed by heating the reaction media globally. These results were used to obtain the linearization plot of the Arrhenius equation to directly correlate temperatures with initial rates of substrate bioconversion. Using the Arrhenius plot obtained for each hybrid (below the inactivation temperature), it was then possible to correlate increases observed in enzyme activity with the local temperature value that the enzyme is sensing while AMF is applied. As the global temperature of the reaction mixture was also monitored while the AMF is applied, it is possible to know if this increase in activity is due to the global heating of the media, or the local heating of the enzyme environment within agarose pores.

1.2.2.1)- Homogenous vs heterogenous hybrids of *Thermomyces lanuginosus* lipase.

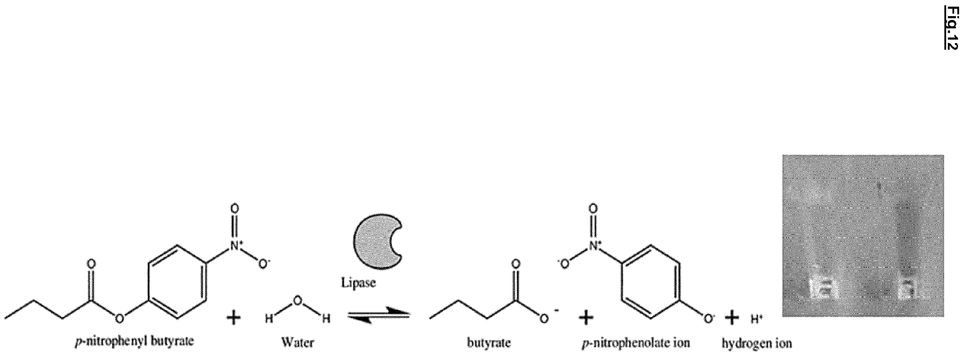

*Thermomyces lanuginosus* lipase (TLL) is monomeric (55 kDa) with an optimal temperature ($T_{OPT}$) of 45 °C. TLL was immobilized on homogenous and heterogenous mHs. TLL lipase activity was determined spectrophotometrically using p-nitrophenyl butyrate (p-NPB) as the substrate. The release of 4-nitrophenolate anion as product (yellow at pH values above its pKa =7.08 at 22 °C), was monitored at a wavelength 384 nm during the enzymatic hydrolysis of the substrate (Figure 12).

After the immobilization of CSIC10e on agarose porous micro beads using the protocol for fast or slow immobilization, their activation with glutaraldehyde and finally the binding of TLL on immobilized particles surface was carried out (Figure 13A). The obtained mHs with a differential spatial location of the MNPs were characterized in terms of optimal activity temperature and kinetic thermal stability at 45 °C. The profile of activity versus temperature allowed to define the optimal temperature of the immobilized protein on both types of mHs, as represented in Figure 13. For these experiments, the mHs were diluted in the reaction media and incubated for 5 minutes at the desired temperature in a thermoblock, the activity was then measured at 348 nm by determining the rate of p-nitrophenolate (p-NP) formation. Besides, for both heterogeneous and homogeneous TLL hybrids, the optimal temperature of the enzyme bound to the MNPs is like that of the free enzyme: around 40-45°C (Figure 13B1 and 13C1). This means that no significant rigidification of the enzyme structure happened upon its immobilization on the surface of the MNPs previously integrated within the agarose beads. In Figure 13B2 and 13C2 the kinetic enzyme stability at 45°C is represented, confirming a good remaining activity during the first hour (around 80%) of incubation for both heterogenous and homogeneous TLL hybrids.

Thus, using the specific activity assay conditions for TLL, both mHs were subjected 5 minutes to an AMF of 772 kHz and 30mT, 309 kHz and 16 mT and 146 kHz and 30mT. The activity of the mHs was measured by determining the units of enzyme (EU) from the slope obtained after plotting the absorbance measured after 0, 2.5 and 5 minutes using a spectrophotometer. The extent of the increase in activity triggered by AMF application could be directly correlated with the local temperature that is sensed by the enzyme. The correlation between enzyme activity and local temperature is studied using the Arrhenius plot, which is obtained using the Arrhenius equation and the data obtained from the optimal temperature profile of each mHs. As explained before, the optimal temperatures profiles are obtained by using the same activity assay conditions (incubation time, reactive mixture composition) than when applying AMF but incubating the mHs at different temperatures using a thermoblock as global heating source.

Figure 14 and 15 showed the changes in activity observed due to the application of the different selected AMF conditions upon heterogenous and homogeneous TLL@mHs. In both cases, a significant increase of the activity was observed compared with activity of TLL@mHs at room temperature without AMF being applied (Figure 14A/15A). Using the Arrhenius plot obtained for each hybrid (Figure 14B/15B), it was possible to correlate this increase in enzyme activity with the local temperature value that the enzyme is sensing while AMF is applied. As the global temperature of the reaction mixture was also monitored while its application (Figure 14C/15C), it is possible to know if this increase in activity is due to the global heating of the media, the local heating of the enzyme environment within agarose pores or a mixture of both phenomena. Interestingly, a different behaviour is observed depending on the spatial distribution of the MNPs within the agarose beads. In the case of the heterogenous mHs, a significant heating of the reaction media was observed even if the concentration of mHs used for these assays is much lower than the one used for the SAR determination. Subtracting the global temperature reached by the reaction media from the local temperature inferred from the observed raise in enzyme activity, it is possible to differentiate the increase in activity caused directly by an increase in the local temperature in the nano/microenvironment of the enzyme. Thus, even at the SAR conditions applied with the maximum frequency and field intensity (772 kHz/30 mT), local heating triggered by AMF is happening. The higher increase in enzyme activity due to a local heating effect is higher when using AMF conditions with lower applied frequency and/or field intensity. This could be possible explained due to an overheating of the enzyme at the maximum AMF conditions used which could cause its partial denaturation. In the case of the homogenous hybrids, a global raise on the temperature of the reaction media was not observed. This, the net increase of enzyme activity over the global temperature of the media is only caused by local heating triggered by AMF. In this case, the increase on the activity reached are much higher than in the case of heterogenous mHs even if the AMF conditions used were the same. This is another clue that overheating of the enzyme micro/nano environment could be the cause of the lower local AMF-triggered enzyme activation observed in the case of heterogenous mHs.

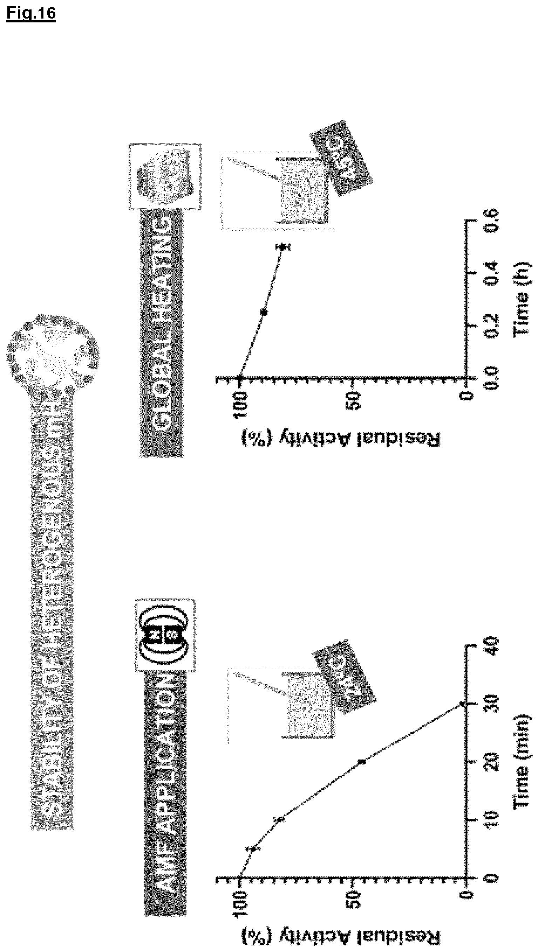

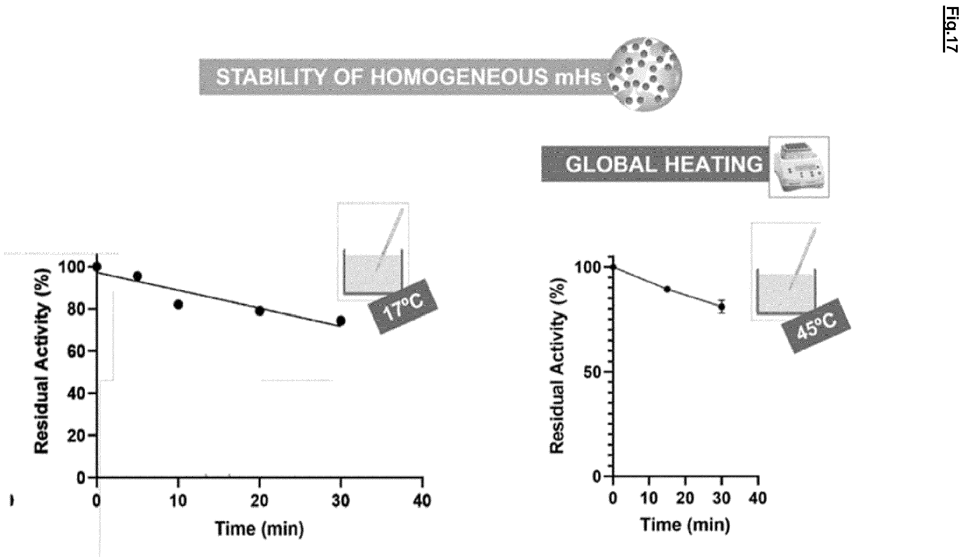

TLL lipase has an optimal temperature around 45°C thus is not considered a thermophilic enzyme thus having moderate thermal stability at high incubation temperatures. Thus, to confirm if the local temperature inferred is a result exclusively of the increase in enzyme activity of the results of mixture between enzyme activation/denaturation process, a stability assay was performed in which the remaining activity of both mHs was assessed after AMF application at the highest frequency/intensity assayed and compared with the stability of the mHs when incubated at 45°C using a thermoblock as global heating source. As it could be observed in Figure 16 and 17, in the case of heterogenous mHs, a clear overheating of the enzyme occurred during AMF application as mHs are completely inactivated after 30 min of AMF application even if the global temperature of the reaction media was 24°C. The local temperature reached at the microenvironment of the enzyme should be higher than 45°C, as more than 80% of the enzyme activity is recovered after incubating the mHs at 45°C for the same length of time. However, it cannot be ruled out that this sharp loss of activity could be also caused by a collapse of the agarose pores due to a melting process AMF-triggered by the high temperatures reached at the microenvironment of the enzyme.

Due to the better results obtained with TLL homogenous mHs, we have focused on the same type of hybrids for other enzymes. As it has been mentioned with this mHs a global raise on the temperature of the reaction media was not observed. Thus, the net increase of enzyme activity over the global temperature of the media is only caused by local heating triggered by AMF. All the applied AMF conditions used caused a significantly increase of the hybrid activity compared to those hybrids that were not subjected to AMF application and were at room temperature during the activity assay. Besides, AMF-enzyme activation is reached while enzyme stability is maintained.

1.2.2.2)- Homogenous hybrids of alcohol dehydrogenase from *Thermus thermophilus*

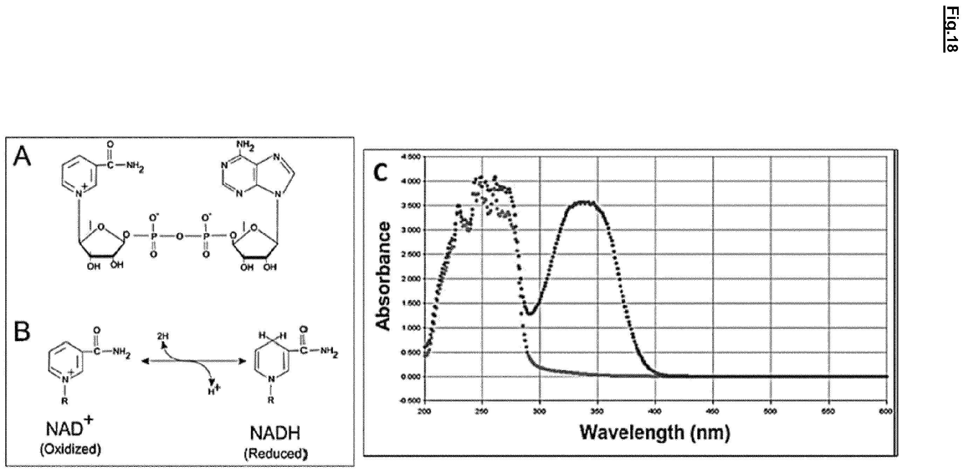

Alcohol dehydrogenase from *Thermus thermophilus* (TtADH) is a thermophilic (TOPT 70 °C) and hexameric protein (196 kDa) involved in diverse metabolic pathways, such as phenylalanine metabolism, butanoate metabolism or benzoate degradation. This enzyme belongs to the large family of NAD(P)H dependent dehydrogenases (EC 1.1.1.X) that catalyze the asymmetric reduction of beta-keto esters and its reverse oxidative reaction with exquisite enantioselectivity. TtADH activity was determined spectrophotometrically following the reduction rate of ethyl acetoacetate (EAA) as substrate at 340 nm by determining the decrease in the absorbance caused by the oxidation of the cofactor nicotinamide adenine dinucleotide (NADH) to the oxidized from NAD+ (Figure 18).

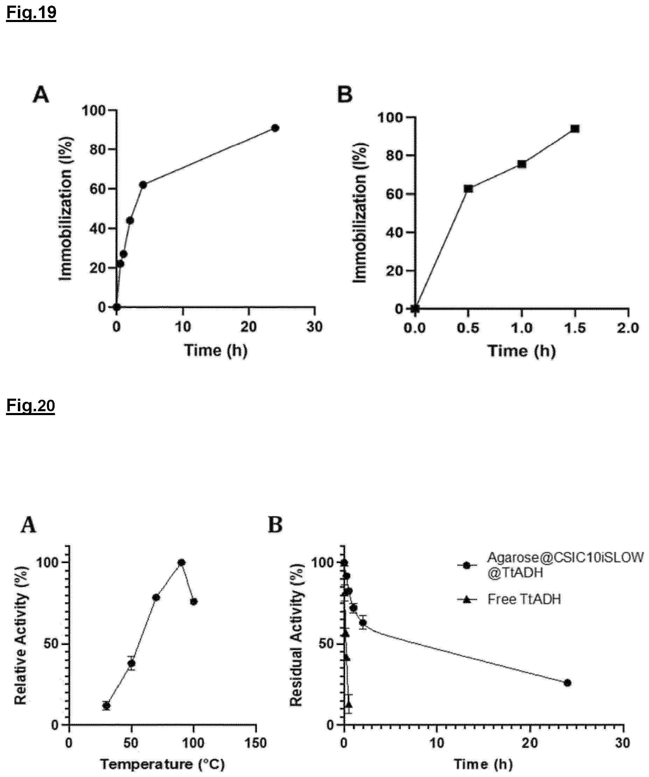

As it could has been observed in Figure 19, the enzyme immobilization was carried out after the activation of the amino groups on the MNPs surface with glutaraldehyde, commonly used as cross-linking agent due to its effectiveness in the immobilization of aminated biomolecules to different types of aminated materials. After optimizing the slow immobilization of CSIC10g MNPs (Figure 19A) on glyoxil-agarose (reaching around 60% of MNPs immobilization after 2 h of incubation), TtADH immobilization kinetics was studied having previously activated the aminated surface of the MNPs overnight with glutaraldehyde (Figure 19B). As it can be observed, 100% of the TtADH offered (0.4 mg/mL) was immobilized after 90 minutes of incubation, being the enzyme activity of the immobilized enzyme around 60% (expressed activity %). Although these results were obtained using CSIC-10g batch of MNPs, similar results were obtained when using CSIC10m batch.

The relation between the reduction rate of ethyl acetoacetate (EAA) versus temperature was determined with the TtADH mHs obtained. The soluble enzyme presents an optimal temperature range of 70°C while that of the immobilized one shifted to 90 °C (Figure 20A). This behaviour is often observed in immobilized enzymes [J. Am. Chem. Soc. 2020, 142, 3463-3471; JFB 2021, 12, 32] as the immobilization process may provoke slight changes in the biocatalyst structure leading to shifts in optimal parameters or stabilization of the 3D structure that impact even on short time activity assays. Indeed, TtADH immobilization within the mHs provided its stabilization (x20 times) being the half-life time at 70 °C of 10 h while only of 30 min for the soluble enzyme (Figure 20B). The thermal stabilization achieved is of great importance in the context of this work in which magnetic heating is the remote stimulus selected for tuning the enzyme activity.

Figure 21 showed the AMF tunability of the microhybrids of TtADH prepared using a homogenous MNPs spatial distribution. To study the AMF-tunability, changes in the initial rate on the reduction of ethyl acetoacetate (EEA) was determined by following the decrease at 340 nm caused by the oxidation of the cofactor nicotinamide adenine dinucleotide (NADH) to its oxidized form (NAD+). Statistical significance of the observed differences were studied comparing the rates obtained upon AMF application with those obtained using the same activity assay conditions but not applying an AMF (No AMF control) (Figure 21A). As it could be observed in Figure 21C, when a field intensity of 30mT was applied, the local temperature that is sensed by the enzyme attached to the MNPs resulted around 60 °C when a frequency of 146 kHz was applied. Increasing the frequency up to 722 kHz, increase the reaction rate of the mHs as if it is incubated at 70°C. However, in both AMF conditions, no change on the global temperature of the reaction media was observed which was maintained at room temperature (RT, 18°C).

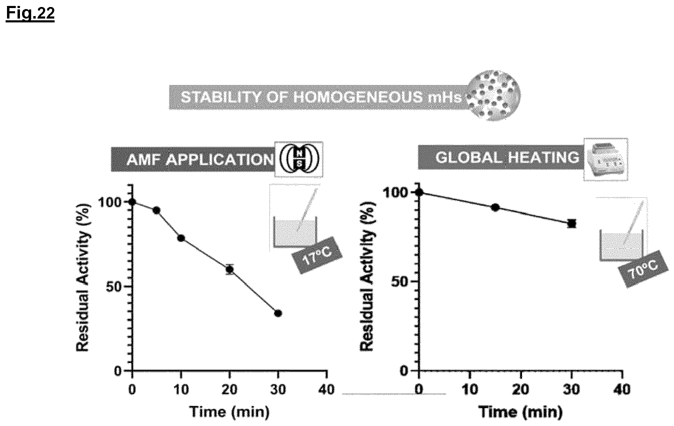

A stability assay confirmed that an overheating of the enzyme occurred while an AMF of 30 mT and 722 kHz is applied, as the AMF-exposed mHs suffered a sharp reduction of their activity even if the global temperature of the reaction media was kept at RT while the AMF was applied (Figure 22). However, when the same study was performed applying the same field intensity (30 mT) but a lower frequency (146 kHz), only 25% of enzymatic activity was lost upon 30 min of AMF application.

From the results obtained, it is possible to conclude that the local heating of an enzyme attached to MNPs that were previously covalently integrated within microporous agarose beads is feasible. Besides, it is also possible to concluded that even if the magnetic heating efficiency of the MNPs covalently integrated within the mHs resulted significantly lower compared with the bare ones, it is possible to trigger high local temperatures like the optimal temperature of TtADH ($T_{OPT}$= 70°C).

1.2.2.3)- Homogenous hybrids of α-amylase from Bacillus licheniformis.

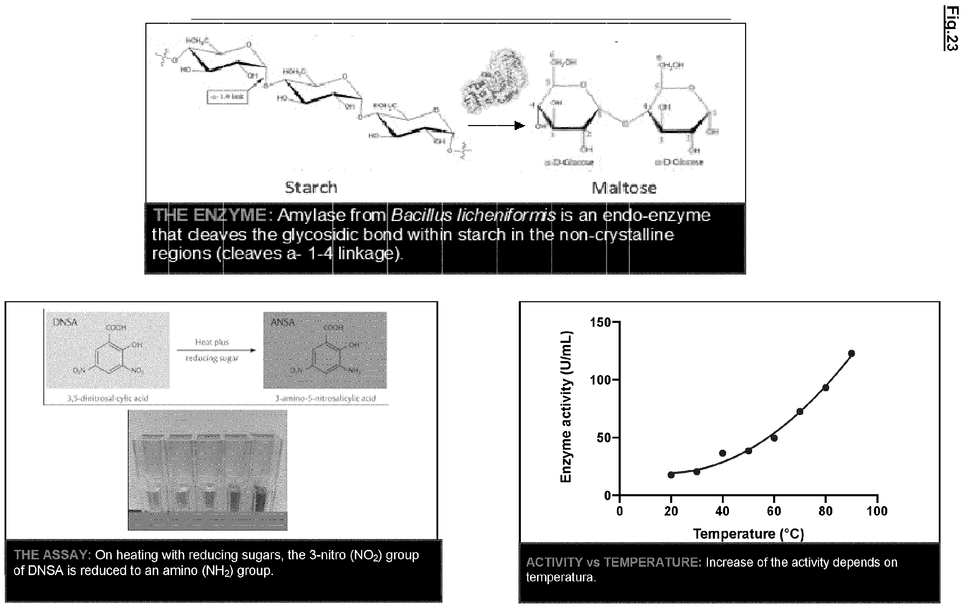

α-amylase from *Bacillus licheniformis* (BIAm) is a monomeric enzyme of 63 kDa, with an optimal temperature of 100°C. It is able to catalyze the degradation of starch into small molecules, such as maltodextrins and maltose being widely applied in the paper industry. A colorimetric assay was performed to follow this reaction during time. The colour of the reagent changes from yellow to orange or red, depending upon the concentration of the reducing maltose present in the mixture (Figure 3.6). In this case, the obtention of a BIAm homogenous mHs was optimized, and its response to the temperature was studied by incubating 2 mg/mL of the hybrid for 15 minutes under stirring. At fixed time points an aliquot of the reaction was withdrawn and analyses with the DNSA assay. From the graph it is possible to observe a strong dependency of the activity from temperature (Figure 23) which is similar to the Temperature profile of the soluble enzyme.

AMF-tunability of the mHs obtained with α-amylase from *Bacillus licheniformis* (BIAm) was also achieved. The activity of the immobilized amylase was studied under an AMF of 351 kHz and 20 mT (Figure 24). An increased activity compared to the NO AMF sample is observed, although not sufficient to reach an activity comparable to the 90°C. Although the global temperature of the reaction media remained unchanged (21°C), using the Arrhenius plot obtained from the Temperature profile of the mHs, it is possible to infer that the enzyme integrated within the hybrid is working as if incubated at a global temperature around 60°C. No activity was detected when agarose hybrid with CSIC-10 immobilized nanoparticles w/o amylase was subjected at any of the condition tested.

1.2)- TUNING MAGNETIC HEATING BY CONTROLLING THE ROTATION CAPACITY OF THE INTEGRATED MNPs.

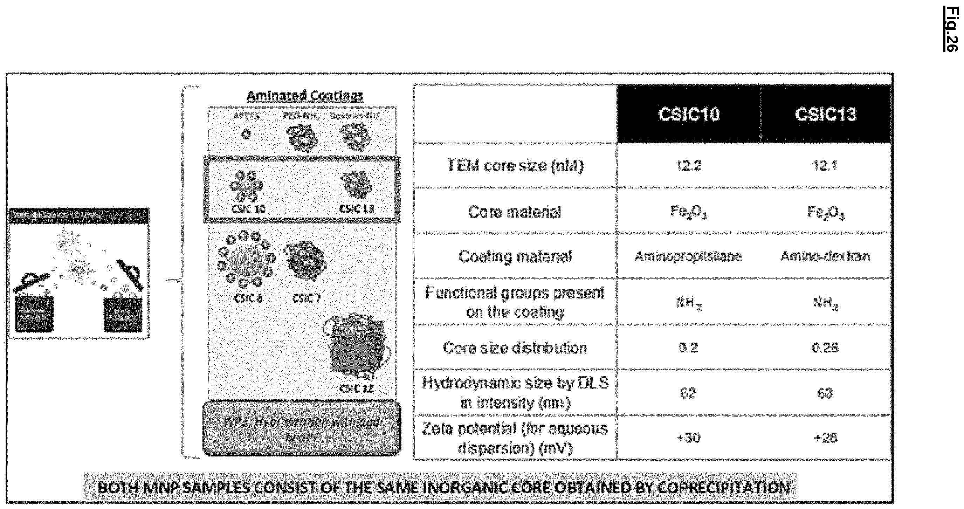

To optimize the obtention of different spatial distribution of the MNPs within the porous beads, an aminated MNPs with amine groups located very close to the MNPs surface was used (CSIC10). Thus, the integration of this MNPs to the agarose beads occurred via very short multipoint covalent bonds. Thus, the rotation capacity of the MNPs should be significantly altered and this could affect the dissipation of heat by Brownian relaxation. Thus, we hypothesized that if we could bind MNPs with the same inorganic core but having exposed amine groups linked via longer spacer arms to the MNP surface, the rotation capacity of the MNPs would be less impeded. Therefore, it should be expected that the heating efficiency will be higher and even lower AMF frequencies could be used to trigger a remote enzyme activation as if its working at its TOPT. For this purpose, the same inorganic core used to prepare CSIC10 MNPs but with amino dextran as coating were used to prepare homogenous TtADH microhybrids This MNPs have been named as CSIC13 (Figure 25). Although having a different coating their physicochemical properties were very similar to CSIC10 (Figure 26).

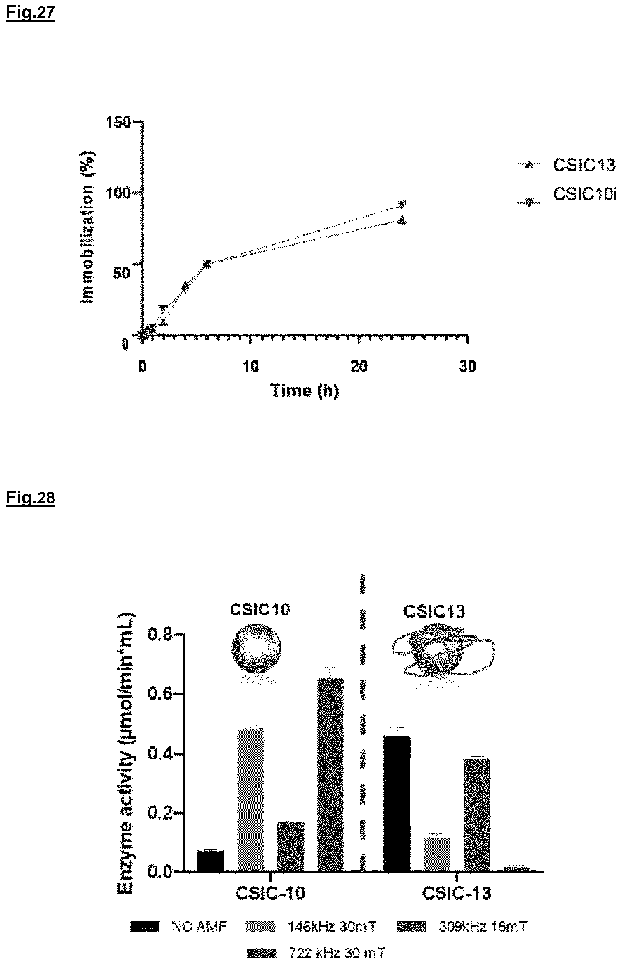

Their immobilization in glyoxil-agarose beads activated with 52,4 μmol aldehyde/mL was carried out using the same incubation conditions that were optimized with CSIC10i batch of NPs to achieve a slow immobilization kinetics. The percentage of immobilization was calculated from the decrease in absorbance at 400 nm. Their kinetic of immobilization of was compared with CSIC10i. As it could be observed in Figure 27, CSIC 13 MNPs has a slow immobilization profile similar to CSIC10i, being only 50% of the applied MNPs immobilized after 6 h and 100% after overnight incubation. Thus, a homogenously distribution of MNPs was also achieved.

Due to the better enzyme local heating performance of homogeneous mHs obtained with CSIC10 MNPs, AMF-triggered local activation assays have been performed with the obtained CSIC13@hybrids after their functionalization with TtADH using glutaraldehyde as previously optimized. Even if a homogenous distribution of CSIC13 MNPs is expected, a global raise on the temperature of the reaction media was observed. In the case of CSIC10, this effect was only observed with heterogenous mHs hybrids. Besides, unlike what was observed with CSIC10 homogenous mHs, in this case any AMF condition applied caused the loss of TtADH activity compared with the mHs that were not subjected to AMF and incubated at room temperature during the activity assay (Figure 28). Indeed, when using the highest combination of frequency and field intensity a complete inactivation of the mHs was observed even if the global temperature of the reaction media was only 26 °C. As we have observed previously, being TtADH a thermophilic enzyme (Topt 70 °C), the stability of the enzyme is good (more than 95% of remaining activity) even after 5 min of incubation at 70 °C.

These results demonstrate that even having similar SAR values, the attachment of aminated-MNPs to the agarose thick fibers through longer spacers could allow tuning the local temperature sensed by the enzyme attached to their surface. It seems that the higher magnetic efficiency of CSIC13 could generate a large increase in temperature withing the porous microstructure of the beads even if the global temperature of the reaction media (macroscopic temperature) is not largely affected. If less amount of MNPs is bound, this microscopic heating should be reduced. To test this, we decrease the density of aldehyde groups in the agarose beads, and thus perform the binding of CSIC13 MNPs in glyoxyl-agarose beads activated with 15 μmol aldehyde/mL was carried out following the same workflow than the one used for the immobilization of CSIC10 MNPs, reaching a SLOW immobilization yield of 29% after 30 hours of incubation at 4°C in HEPES pH 7.5, 0.1% cetyltrimethylammonium bromide (CTAB) and 50mM ethylenediamine (EDA), and being the applied amount of NPs of 2mg Fe/g agarose. Once the MNPs were integrated withing the agarose beads, TtADH was covalently attached to their surface by using glutaraldehyde as previously optimized with CSIC10 MNPs (TtADH 4mg/mg Fe bound to the agarose beads). Then, AMF-activation studies have been performed.

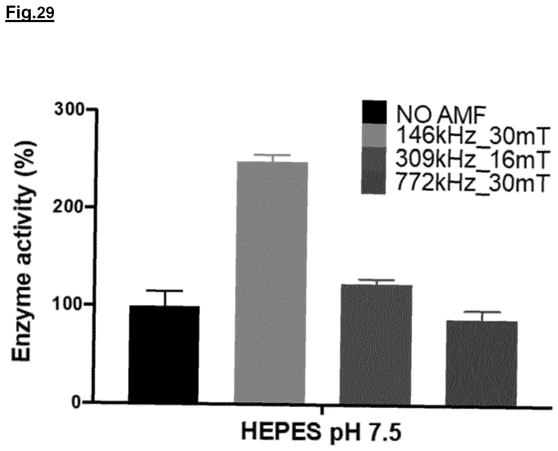

A global raise on the temperature of the reaction media was not observed in any of the different AMF settings that were applied. As it could be observed in Figure 29, in this case, a significant TtADH activation was observed when an AMF of 146 kHz and 30 mT was applied with respect to the EAA reduction rate obtained using the same activity assay conditions but not applying an AMF (No AMF control). However, a lower AMF-triggered enzyme activation is observed when increasing the frequency (772 kHz + 30 mT) of the AMF field even if its intensity was lowered (309 kHz +16 mT). This reduced activation when increasing the frequency/intensity of the AMF applied could be related to overheating of the enzyme leading to its inactivation due to thermal denaturation of its 3D structure. Indeed, when applying the highest combination of frequency and field intensity (772 kHz and 30 mT) for 30 min, the complete inactivation of the immobilized enzyme was observed.

Thus, these results are in line with the ones obtained when a higher amount of MNPs were bound when using agarose beads with a larger amount of aldehyde groups (Figure 28).

The binding of a mesophilic enzyme was carried out using the aldehyde groups that remains free after MNPs covalent attachment to the porous beads, to characterize better heat diffusion at the microscopic level. This way, although the enzyme is not attached to the MNPs surface (CSIC10 or CSIC13), it is very sensitive to an increase of the temperature of its microenvironment and therefore it could act as heat probe. *Candida Bondii* formate dehydrogenase (FDH) was used as the mesophilic enzyme. Being its optimal temperature of 30°C, it is extremely sensitive to its exposure to higher temperatures than 30°C which trigger its denaturation. Thus, instead of immobilizing the enzyme on the surface of the previously integrated MNPs (as it was done with the thermophilic TtADH), the FDH was co-immobilized directly onto the agarose beads taking advantage of the same chemistry used to bind the MNPs: multipoint chemical bonding via amine groups of its surface and the remaining aldehyde groups of the agarose beads (Figure 30).

The co-immobilization workflow consisted of first activating the agarose beads with aldehyde groups. Then the MNPs were immobilized using immobilization conditions previously optimized to ensure a homogeneously distribution of the MNPs. Without reducing the Schiff's bases formed between the amine groups of the MNPs and the aldehydes of the scaffold, after washing the beads, FDH was immobilized using incubation conditions that also promotes its multipoint binding to the scaffold via its amine groups. Once both imine bonds (from MNPs or FDH with the scaffold) were formed, a reduction step using sodium borohydride as reductant allows transforming these reversible Schiff bases into irreversible secondary amine bonds. In the case of co-immobilization with CSIC10 MNPs, agarose containing 52$\mu$mol aldehyde/mL was used, while in the case of CSIC13 MNPs the mHs obtained with a lower amount of aldehydes (15$\mu$mol/mL) have been selected as less overheating of the TtADH attached to the MNPs surface was observed (Figure 29). Below, is a summary of the co-immobilization conditions of MNPs (ensuring a homogenous distribution of the MNPs) and FDH for each of the MNPs tested:

- Agarose 2BCL, glyoxyl activated (52$\mu$mol/mL) in which CSIC10 nanoparticles were immobilized (2mg Fe/g agarose) in 100mM bicarbonate pH8.0, 0.1% CTAB, 50mM EDA at 4°C, with an immobilization yield of 94% after 24 hours. FDH was immobilized on agarose beads surface (1mg FDH/g agarose) in 50 mM bicarbonate pH10 at 4°C for 20h with an immobilization yield of 98%.

- Agarose 2BCL, glyoxyl activated (15$\mu$mol/mL) in which CSIC13 nanoparticles were immobilized (2mg Fe/g agarose) in HEPES pH8.0, 0.1% CTAB, 50mM EDA at 4°C, with an immobilization yield of 51% after 24 hours. FDH was immobilized on agarose beads surface (1mg FDH/g agarose) in 50 mM bicarbonate pH10 at 4°C for 20h with an immobilization yield of 59%.

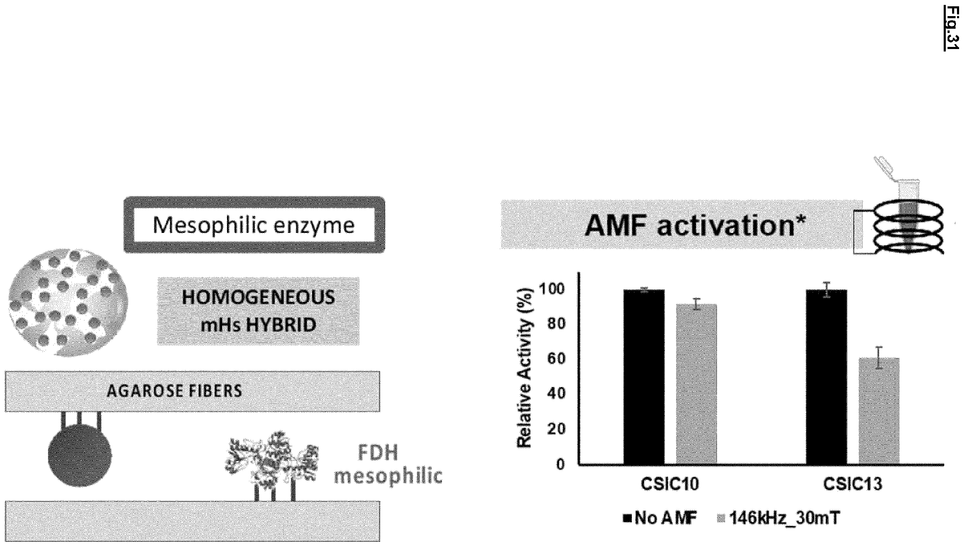

With both hybrids the activity of the immobilized FDH was measured applying or not an AMF of 146 kHz and 30mT for 5 minutes and results are reported in Figure 31. It is important to highlight that the global temperature of the reaction media remained unaltered (20 ± 0.4 °C) during AMF application. Unlike the thermophilic enzyme, FDH is not immobilized on the surface of the MNPs acting as nanoheaters, but rather is bound to the agarose fibers. Thus, if the microenvironment near the enzyme is not heated up due to the activation of the co-immobilized MNPs due to AMF application, it is expected that the activity measured with and without AMF application to be the same. However, as the FDH is very sensible to temperature, it is expected a lower activity if an overheating of the enzyme microenvironment occurs while measuring its activity while an AMF is applied.

From the results obtained it could be observed a non-significant decrease of the enzyme activity when CSIC10 MNPs were co-immobilized with the FDH, even if a higher amount of MNPs were integrated within the mHs. However, those mHs exposed to AMF while measuring their enzyme activity prepared with CSIC13 MNPs present a significant lower conversion rate of the substrate (around half of the activity) compared to those not subjected to AMF. These results, also support that CSIC13 MNPs has a higher magnetic heating efficiency even if the inorganic core is the same to CSIC10 and less MNPs have been integrated within the mHs structure. This higher efficiency led to a higher overheating of the reaction media at the microenvironment of the co-immobilized enzymes molecules within the microbeads (microscopic temperature) although the global temperature of the reaction media remained however unaltered (macroscopic temperature).

Thus, different complementary assays have been conducted that support that the way MNPs are fixed to the agarose fibers reducing or promoting their AMF-triggered rotation (trough multipoint short bonds or flexible spacers) could also be a way of tuning the magnetic heating efficiency of the MNPs. This additional strategy could be used in combination with other strategies that we have showed that also allow tuning their magnetic heating efficiency as it is the control of their distribution within the agarose beads and/or tunning the AMF conditions applied.

## 2) MICROHYBRIDS: INCREASING VERSATILITY WITH RESPECT TO THE METHODOLOGY USED TO ATTACH ENZYMES ON THE INTEGRATED MNPs.

To increase the versability with respect to the enzyme functionalization of the mHs to the MNPs integrated within the agarose beads, a different conjugation chemistry was optimized. The strategy initially proposed (Figure 2) only allowed the covalent binding of enzymes to the MNPs that were previously integrated via amine groups present at the surface of the enzyme of interest by using glutaraldehyde as crosslinker agent. Thus, to exploit the recombinant His-tagged/His-cluster tagged enzyme variants obtained during the project to control enzyme orientation, a protocol for the introduction of the His-tagged chelator agent NTA-Co was developed.

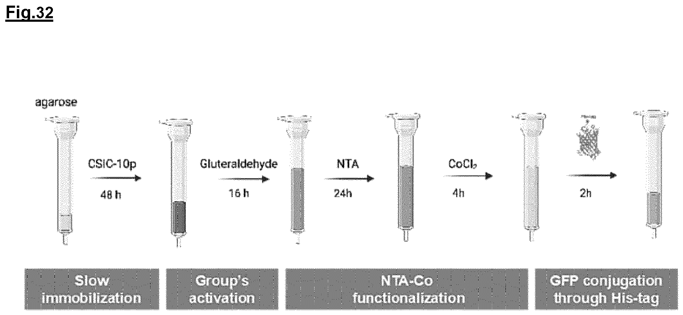

For this purpose, the previously optimized protocol for the conjugation of the protein via its amine groups was slightly modified. First, the aminated nanoparticles were immobilized on the microbeads via glyoxyl groups. Subsequently, the remaining amine groups of the MNPs were activated with glutaraldehyde, but instead of directly adding the enzyme of interest, a nitril acetic acid derivative containing a reactive primary amine group (NTA) was introduced on the nanoparticle surface. Finally, samples were incubated with a 100 mM $CoCl_2$ solution to chelate the metal ion into the NTA moiety, and thus introducing to the mHs the ability to bind His-tagged proteins by metal affinity chelation (Figure 32).

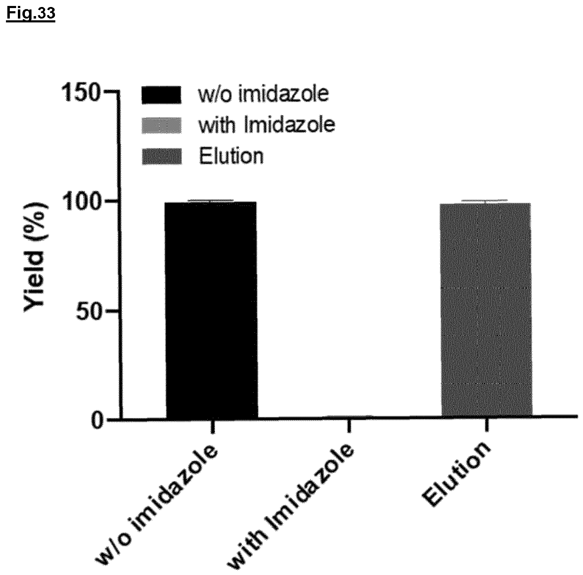

To validate this functionalization chemistry, the his-tagged green fluorescent protein (GFP) was used as model for probing the feasibility of its specific immobilization. For this, the NTA-Co@mHs obtained were incubated for 2 h at room temperature under stirring in presence or absence of 0.5M imidazole and the recombinant protein. Figure 33 shows the specificity of the conjugation. Indeed, no immobilization was observed when the GFP was incubated with the mHs in the presence of imidazole. On the same way, to confirm that no further unspecific interaction with the MNPs was occurring after the his-tagged specific binding, the protein once bounded was eluted from the mHs by incubating the hybrids with 0.5 M imidazole. A quantitative elution of the sample was reached.

These results suggest the possibility to explore this specific protocol for an oriented conjugation of all the His-tagged or His-clusters enzyme variants prepared during the project, which would allow studying the effect of protein orientation in. the energy transference process between MNPs and enzyme molecules.

## DETAILED IMMOBILIZATION PROTOCOL

## A) PROTOCOL FOR MNPs BINDING TO AGAROSE BEADS

Briefly, the MNPs were sonicated (30 sec approx.), and then added in a glass flask with the immobilization buffer (Note1), in a final concentration of aup to 2mgFe/g wet agarose The solution is sonicated for 30sec more before mixing with the agarose.

So, 0,5g of agarose support were weighted in a glass flask and the MNPs solution was adde. The reaction is carried under stirring, at the temperature that the needed conditions to obtain a slow/fast immobilization. Periodically, samples of the supernatant were withdrawn to determine the immobilization rate. Samples were diluted in 1.8 mL of sodium bicarbonate buffer at pH 8.5 to measure the absorbance at 400 nm by spectrophotometry (Jasco V-730 spectrophotometer_ Cary). When the immobilization was 100%, the **preparation was reduced** using 1mg/mL of NaBH4 for 30 minutes. Finally, the mixture was washed with 50 mL of sodium bicarbonate buffer 25 mM at pH 8.5 and filtered.

NOTE1: It has been called immobilization buffer each preparation used for the immobilization of MNPs. Its composition depends on the immobilization rate needed (fast or slow).

## Slow immobilization

For achieving a homogenous integration, the immobilization is carried out at 4 °C at a pH 8.5, using: i) 0.1 M Sodium bicarbonate, ii) 2BCL agarose beads activated with 52 $\mu$mol/mL aldehyde groups, iii) Schiff's base stabilizing agents (eg. Sodium borohydride, DTT, mercaptoethanol), iv) aminated molecules as competitors for agarose aldehyde's groups to slow down the kinetics of immobilization, in this case 50 mM ethylenediamine and v) surfactants to improve colloidal stability of aminated MNPs over long incubation times (>24 h). After optimization, homogenous MNPs covalent integration was carried out at 4 °C in 0.1 M Na-bicarbonate buffer pH 8.5 or 0.1M HEPES pH 7.5, 0.1% CTAB and 50 mM ethylenediamine.

**Fast immobiization**

For achieving an heterogenous integration, the immobilization is carried out at room temperature at pH ranging between 8-10 (being 10 usually preferred binding pH) using a broad range of buffers (Na-bicarbonate, Na-borate, CAPS, etc) and a high density of aldehyde groups at the agarose beads (ranging from 25-60) and MNPs with a range of amino groups from 40 to 84 NH2/mgFe. After optimization, the heterogenous MNPs covalent integration was carried out at 25 °C in 0.01 M Na-borate buffer pH 8.0 or 0.1 M Na-bicarbonate buffer pH 10.0 and 0.1% CTAB;

**B) PROTOCOL FOR ENZYME BINDING TO THE MNPS INTEGRATED TO THE AGAROSE BEADS.**

**Glutaraldehyde activation of MNPs Amine groups**

Briefly, 2g of hybrid composites was mixed with 2.5 mL of 0.2 M sodium phosphate buffer pH 7 and 3.4 ml 25% glutaraldehyde. This mixture was incubated for 12-14 h at room temperature protect from the light. Then, the hybrids are washed with the phosphate buffer for 3 times, using a vacuum pump.

**Enzyme Immobilization Protocol onto previously integrated MNPs**

A solution of the targeted enzyme in 25mM sodium bicarbonate buffer pH 8.5 was prepared and mixed with agarose hybrid with MNPs, modified with glutaraldehyde. Afterward, the sample was incubated at 25 °C with gentle stirring. To control the immobilization rate, an aliquot of supernatant was taken at different times (time 0, 30, 60 and 90 min). The supernatant was analysed both with Bradford assay and activity assay. When the immobilization is achieved, the solution is filtered in a syringe, and the hybrid washed 3 times with 25mM Phosphate buffer pH7.5.

**SUMMARY OF MAIN ACHIEVEMENTS:**

1. Obtention of microhybrids with different spatial location of MNPs covalently integrated to the microbeads used for their preparation (homogenous and heterogenous@MNPs mHs), having also identified those key parameters to ensure their reproducible production.

2. Optimization different techniques to analyze the different spatial distribution of the MNPs withing the microbeads: confocal microscope imaging, SEM analysis of ultrathin slices of embedded hybrids in resin, TEM, SAR determination.

3. Optimization of two different functionalization chemistries to achieve the site-directed binding of the thermophilic enzyme to be AMF-tuned onto the surface of the MNPs previously integrated within the mHs: i- via amine groups of the enzyme (using glutaraldehyde as crosslinker), or ii)- via His-tags or His-clusters present on recombinant enzymes (using metal affinity binding).

4. Versatility of the developed enzyme conjugation chemistries of mHs using different enzymes (TtADH, TLL lipase, BLAm amylase).

5. AMF-triggered enzyme activation observed with the microhybrids (mHs) obtained using TLL, TtADH and BIAm being the local temperature that is sensed by them tunable by: i) the spatial distribution of the MNPs within the beads structure, ii) the AMF conditions applied, and iii) the attachment of the MNPs to the microbeads through long or short spacers.

**Claims**

1.   A process for the production of a system comprising:

a) providing a porous scaffold, wherein the porous scaffold comprises or is activated to provide on its surface hydroxyl, carboxyl, carbonyl, amino, thiol, azide, or alkyne functional groups capable of covalently binding MNPs to said porous scaffold,
b) providing at least a population of magnetic nanoparticles (MNPs), **characterized in that** the MNPs have a magnetic anisotropy capable of dissipating local thermal energy on their surface upon application of an external alternating magnetic field (AMF), wherein the MNPs comprise or are activated to provide on their surface hydroxyl, carboxyl, carbonyl, amino, thiol, azide, or alkyne functional groups capable of covalently binding the porous scaffold of step a), and
c) contacting the MNPs with the porous scaffold under suitable conditions for the immobilization of the MNPs to the scaffold by means of covalent bonds,

wherein steps a) and b) can be performed in any order.

2.   The process according to claim 1, further comprising a step d) of contacting the MNPs with at least one biomolecule under suitable conditions for the immobilization of the biomolecule to the MNPs by means of covalent bonds or affinity metal interactions.

3. The process according to claim 2, wherein the at least one biomolecule is an enzyme, wherein said enzyme is immobilized to the MNPs by means of covalent bonds, and wherein the covalent bonds are preferably carried out by reacting an amino group present on the surface of the MNP with a carboxyl group comprised in the enzyme.

4. The process according to any one of claims 2 or 3, wherein the functional groups present on the surface of the MNPs and capable of binding to the biomolecule are separated from the surface of the MNPs by a distance of more than 1 nm by using a linker molecule.

5. The process according to any one of claims 2 or 3, wherein the functional groups present on the surface of the MNPs and capable of binding to the biomolecule are separated from the surface of the MNPs by a distance of equal or less than 1 nm by using a linker molecule.

6. The process according to any one of the previous claims, wherein the porous scaffold comprises agarose beads, wherein the agarose beads have a size of 50-200 $\mu$m and a pore size of between 50-200 nm.

7. The process according to any one of the previous claims, wherein previously or subsequently to step c), the porous scaffold is contacted with at least one biomolecule under suitable conditions for the immobilization of the biomolecule to the surface of the scaffold by means of covalent bonds or affinity metal interactions.

8. The process according to any of the previous claims, wherein the immobilization of step c) is a homogeneous or heterogenous immobilization.

9. The process according to claim 8, wherein the porous scaffold comprises agarose microbeads and is coated and/or activated to provide on its surface carboxyl functional groups, wherein the MNPs are coated and/or activated to provide on their surface amino functional groups, and wherein the immobilization of step c) is a homogeneous immobilization carried out at a temperature of less than 10°C, at a pH ranging between 7-9 and during periods of between 24h and 72h, and using:

    i) a buffer,
    ii) aldehyde agarose beads having from 4 to 52 $\mu$mol of aldehydes groups/wet gram of agarose,
    iii) Schiff's base stabilizing agents,
    iv) aminated molecules as competitors for agarose aldehyde's groups to slow down the kinetics of immobilization; and
    v) surfactants; and
    vi) MNPs with a range of amino groups from 159 to 352 $NH_2$/mg Fe,

wherein the reaction is carried according to the following steps:

    a) adding the agarose beads to the aminated MNPs dispersions and incubate the mix for at least 24 h up to 72h at an incubation temperature lower than 10°C,
    b) reducing the Schiff's bases to secondary amino groups and preferably the remaining aldehyde groups into hydroxyl ones by adding a suitable reducing agent such as sodium borohydride,
    c) optionally, washing the MNPs immobilized to the agarose beads,

wherein preferably, after MNPs immobilization, derivatives are reduced with a suitable reducing agent such as sodium borohydride in order to reduce Schiff's bases to secondary amino groups and to optionally reduce remaining aldehyde groups into hydroxyl ones, and wherein after MNPs immobilization, the MNPs are preferably activated to functionalize their surface with at least one biomolecule, by covalent bonds or metal affinity.

10. The process according to claim 8, wherein the porous scaffold comprises agarose microbeads and is coated and/or activated to provide on its surface carboxyl functional groups, wherein the MNPs are coated and/or activated to provide on their surface amino functional groups, and wherein the immobilization of step c) is a heterogenous immobilization carried out at a temperature of between 25-20°C, pH ranging between 8-10, and during periods of time between 30 minutes to 1h, and using a suitable buffer, wherein the reaction is carried according to the following steps:

    a) adding the aldehyde agarose beads to the aminated MNPs dispersions and incubate for at least 30 minutes up to 1 h at a temperature of between 25-20°C,
    b) reducing the Schiff's bases to secondary amino groups and optionally the remaining aldehyde groups into hydroxyl groups by adding a suitable reducing agent such as sodium borohydride,
    c) optionally washing the MNPs immobilized to the agarose beads,

wherein preferably, after MNPs immobilization, derivatives are reduced with a suitable reducing agent such as sodium borohydride in order to reduce Schiff's bases to secondary amino groups and to reduce remaining aldehyde groups into hydroxyl ones, and wherein after MNPs immobilization, the MNPs are preferably activated to functionalize their surface with at least one biomolecule, by covalent bonds or metal affinity.

11. The process of any of the previous claims, wherein the MNP have an iron oxide core and are selected from the group consisting of:

    i) having an average particle diameter of between 8-15 nm, being coated APTES and having a magnetic anisotropy of 34 $\pm$ 50%, preferably 27 mT $\pm$ 25% , or
    ii) having an average particle diameter of between 8-15 nm, being coated aminated-dextran (DEX) and having a magnetic anisotropy of 34 $\pm$ 50%, preferably 27 mT $\pm$ 25% .

12. The system obtained from the process of claims 1 to 11.

13. A system comprising:

    a) a porous scaffold, wherein the porous scaffold is **characterized by** comprising or being activated to provide on its surface at least one functional group capable of covalently binding MNPs to said porous scaffold, wherein said at least one functional groups are selected from the list consisting of hydroxyl, carboxyl, carbonyl, amino, thiol, azide, or alkyne functional groups.
    b) a population comprising MNPs, **characterized in that** the MNPs have a magnetic anisotropy capable of dissipating local thermal energy on their surface upon application of an external alternating magnetic field (AMF), wherein the MNPs are further **characterized by** comprising or being activated to provide on their surface at least one functional group capable of covalently binding said MNPs to the porous scaffold of a), wherein said at least one functional groups are selected from the list consisting of hydroxyl, carboxyl, carbonyl, amino, thiol, azide, or alkyne groups.

14. *In vitro* use of the system as defined in any of claims 2 to 11, or the system of claim 12 or 13, for carrying out at least an enzymatic reaction.

15. A method for carrying out an enzymatic reaction, the method comprising the steps of:

    a) carrying out the process as defined in any of claims 2 to 11, or providing the system of claim 12 or 13, wherein the at least one biomolecule immobilized on the surface of the MNPs is an enzyme, and
    b) applying one or more external AMFs to the system of a) to produce the activation of the at least one enzyme functionalized on the MNPs so that the enzymatic reaction is carried out.

Fig. 1

**Fig. 2**

**Fig. 3**

**Fig. 4**

FAST IMMOBILIZATION

- 1 mg$_{Fe}$/mL
- 10 mM Borate buffer pH 8.0
- 0.1% CTAB
- Room temperature

CSIC10e

SLOW IMMOBILIZATION

- 2 mg$_{Fe}$/mL
- 0.1 M Sodium bicarbonate buffer pH 9.0
- 0.1% CTAB, 50 mM Ethylenediamine
- 4°C

Fig. 5

**Fig. 6**

**Fig.7**

**Fig.8**

**Fig.9**

**Fig.10**

Fig.11

EP 4 397 743 A1

Fig.12

Fig.13

**Fig.14**

Fig.15

**Fig.16**

**Fig.17**

**Fig.18**

**Fig.19**

**Fig.20**

**Fig.21**

A) *Rate of EAA reduction (magnetic heating)*

B) **Temperature Profile (global heating)**

C) **AMF triggered activity (magnetic heating)**

**Fig.22**

Fig.23

EP 4 397 743 A1

Starch

Maltose

**THE ENZYME:** Amylase from *Bacillus licheniformis* is an endo-enzyme that cleaves the glycosidic bond within starch in the non-crystalline regions (cleaves a- 1-4 linkage).

DNSA

$COOH$

$OH$

$O_2N$ $NO_2$

Heat plus reducing sugar

ANSA

$COOH$

$OH$

$O_2N$ $NH_2$

3,5-dinitrosalicylic acid

3-amino-5-nitrosalicylic acid

**THE ASSAY:** On heating with reducing sugars, the 3-nitro ($NO_2$) group of DNSA is reduced to an amino ($NH_2$) group.

Enzyme activity (U/mL)

Temperature (°C)

**ACTIVITY vs TEMPERATURE:** Increase of the activity depends on temperatura.

Fig.24

Fig.25

## Fig.26

| | | CSIC10 | CSIC13 |
|---|---|---|---|
| | TEM core size (nM) | 12.2 | 12.1 |
| | Core material | $Fe_2O_3$ | $Fe_2O_3$ |
| | Coating material | Aminopropilsilane | Amino-dextran |
| | Functional groups present on the coating | $NH_2$ | $NH_2$ |
| | Core size distribution | 0.2 | 0.26 |
| | Hydrodynamic size by DLS in intensity (nm) | 62 | 63 |
| | Zeta potential (for aqueous dispersion) (mV) | +30 | +28 |

BOTH MNP SAMPLES CONSIST OF THE SAME INORGANIC CORE OBTAINED BY COPRECIPITATION

**Fig.27**

**Fig.28**

**Fig.29**

Fig.30

**Fig.31**

AMF activation*

Relative Activity (%)

146kHz_30mT    No AMF

CSIC10    CSIC13

Mesophilic enzyme

HOMOGENEOUS mHs HYBRID

AGAROSE FIBERS

FDH mesophilic

**Fig.32**

Fig.33

Europäisches
Patentamt

European
Patent Office

Office européen
des brevets

## EUROPEAN SEARCH REPORT

Application Number

EP 23 38 2002

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DONG JING ET AL: "Amperometric biosensor based on immobilization of acetylcholinesterase via specific binding on biocompatible boronic acid-functionalized Fe@Au magnetic nanoparticles", JOURNAL OF SOLID STATE ELECTROCHEMISTRY, vol. 16, no. 12, 19 July 2012 (2012-07-19), pages 3783-3790, XP093043938, DE ISSN: 1432-8488, DOI: 10.1007/s10008-012-1812-6 Retrieved from the Internet: URL:http://link.springer.com/article/10.1007/s10008-012-1812-6/fulltext.html> | 1-10, 12-15 | INV. C12M1/00 C12M1/42 |
| Y | * the whole document * * in particular: * * Scheme 1; page 3786 * * page 3785, left-hand column, line 8 – right-hand column, last paragraph * ----- | 11 | |
| Y | D. GOZUACIK ET AL: "Anticancer Use of Nanoparticles as Nucleic Acid Carriers", JOURNAL OF BIOMEDICAL NANOTECHNOLOGY, vol. 10, no. 9, 1 September 2014 (2014-09-01), pages 1751-1783, XP055570132, US ISSN: 1550-7033, DOI: 10.1166/jbn.2014.1935 * section "Magnetic Nanoparticles"; page 1761 * ----- | 11 | TECHNICAL FIELDS SEARCHED (IPC) C12M |
| | -/-- | | |

The present search report has been drawn up for all claims

| Place of search | Date of completion of the search | Examiner |
|---|---|---|
| The Hague | 8 May 2023 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 38 2002

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2011/008797 A1 (ZILCH CHRISTIAN [DE] ET AL) 13 January 2011 (2011-01-13) * the whole document * * in particular: * * paragraphs [0013], [0014], [0036], [0049], [0053] * | 1-15 | |
| A | LEI LANJIE ET AL: "Magnetic biohybrid microspheres for protein purification and chronic wound healing in diabetic mice", CHEMICAL ENGENEERING JOURNAL, ELSEVIER, AMSTERDAM, NL, vol. 425, 6 June 2021 (2021-06-06), XP086805355, ISSN: 1385-8947, DOI: 10.1016/J.CEJ.2021.130671 [retrieved on 2021-06-06] * page 2, left-hand column, last paragraph * * page 2, right-hand column, paragraph 2.1 * * page 3, paragraph 3.1 * | 1-15 | |
| A | EUGENIA TEODOR ET AL: "Hydrogel-magnetic nanoparticles with immobilized l-asparaginase for biomedical applications", JOURNAL OF MATERIALS SCIENCE: MATERIALS IN MEDICINE, KLUWER ACADEMIC PUBLISHERS, BO, vol. 20, no. 6, 22 January 2009 (2009-01-22), pages 1307-1314, XP019680226, ISSN: 1573-4838 * section "Introduction"; page 1307 - page 1308 * * section "2.4 Particle size analysis"; page 1309 * | 1-15 | TECHNICAL FIELDS SEARCHED (IPC) |

-/--

The present search report has been drawn up for all claims

| Place of search | Date of completion of the search | Examiner |
|---|---|---|
| The Hague | 8 May 2023 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 23 38 2002

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 104 977 339 A (UNIV ZHEJIANG OCEAN) 14 October 2015 (2015-10-14) * figure 3 * * paragraphs [0012], [0022] * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 8 May 2023 | Tuynman, Antonin |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

page 3 of 3

## ANNEX TO THE EUROPEAN SEARCH REPORT
## ON EUROPEAN PATENT APPLICATION NO.

EP 23 38 2002

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2023

| Patent document cited in search report | | Publication date | Patent family member(s) | | Publication date |
|---|---|---|---|---|---|
| US 2011008797 | A1 | 13-01-2011 | CA | 2718353 A1 | 24-09-2009 |
| | | | EP | 2110175 A1 | 21-10-2009 |
| | | | JP | 2011517558 A | 16-06-2011 |
| | | | US | 2011008797 A1 | 13-01-2011 |
| | | | WO | 2009115335 A1 | 24-09-2009 |
| CN 104977339 | A | 14-10-2015 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Sensors,* 2015, vol. 15, 2798-281
- *J. Am. Chem. Soc.,* 2020, vol. 142, 3463-3471
- *JFB,* 2021, vol. 12, 32